(19) Europäisches Patentamt
European Patent Office
Office européen des brevets

(11) Veröffentlichungsnummer : **0 132 826**
**B1**

(12) # EUROPÄISCHE PATENTSCHRIFT

(45) Veröffentlichungstag der Patentschrift : **28.09.88**

(51) Int. Cl.⁴ : **C 07 D 239/28**, A 01 N 43/54

(21) Anmeldenummer : **84108700.0**

(22) Anmeldetag : **23.07.84**

(54) **N-(2-Nitrophenyl)-5-aminopyrimidin-Derivate, deren Herstellung und Verwendung.**

(30) Priorität : **25.07.83 CH 4047/83**

(43) Veröffentlichungstag der Anmeldung : **13.02.85 Patentblatt 85/07**

(45) Bekanntmachung des Hinweises auf die Patenterteilung : **28.09.88 Patentblatt 88/39**

(84) Benannte Vertragsstaaten : **AT BE CH DE FR GB IT LI LU NL SE**

(56) Entgegenhaltungen :
**EP-A- 0 013 143**
**DE-A- 2 520 381**
**GB-A- 1 388 825**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Zondler, Helmut, Dr.**
**Oberwilerstrasse 49**
**CH-4103 Bottmingen (CH)**
Erfinder : **Hubele, Adolf, Dr.**
**Obere Egg 9**
**CH-4312 Magden (CH)**
Erfinder : **Nyfeler, Robert, Dr.**
**Bärenfelserstrasse 8**
**CH-4057 Basel (CH)**

(74) Vertreter : **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun.**
**Bräuhausstrasse 4**
**D-8000 München 2 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue N(2-Nitrophenyl)-5-amino-pyrimidin-Derivate der nachstehenden Formel I.

Die Erfindung betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Mikroorganismen, vorzugsweise pflanzenschädigenden Pilzen und Bakterien.

Bei den erfindungsgemässen Verbindungen handelt es sich um solche der allgemeinen Formel I :

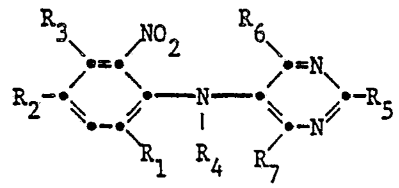

(I)

worin

$R_1$ und $R_2$ unabhängig voneinander für $NO_2$ oder $CF_3$ stehen oder einer von beiden für Wasserstoff steht ;

$R_3$ Wasserstoff oder Halogen bedeutet ;

$R_4$ für Wasserstoff oder die Gruppe —C(O)R' steht, wobei R' unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$, $R_6$ und $R_7$ unabhängig voneinander für Wasserstoff, Halogen, Nitro, Cyano, Rhodano, Mercapto, $C_1$-$C_6$-Alkyl, $C_1$-$C_6$-Haloalkyl, $C_1$-$C_6$-Alkoxy, durch Halogen, Nitro, Cyano, $C_1$-$C_3$-Alkoxy, $C_1$-$C_3$-Alkylthio und/oder $N(C_1$-$C_4$-alkyl$)_2$ substituiertes $C_1$-$C_6$-Alkoxy, $C_3$-$C_6$-Cycloalkoxy, $C_3$-$C_6$-Alkenyloxy, $C_3$-$C_6$-Alkinyloxy, $C_1$-$C_6$-Alkylthio, $C_3$-$C_6$-Alkenylthio, $C_1$-$C_6$-Alkylsulfonyl, $C_1$-$C_6$-Alkylsulfoxyl, $C_3$-$C_6$-Alkenyl, $C_3$-$C_6$-Haloalkenyl, $C_3$-$C_6$-Alkinyl, $C_3$-$C_6$-Haloalkinyl oder für die Gruppe $N(C_1$-$C_4$-alkyl$)_2$ stehen und $R_5$ zusätzlich eine unsubstituierte oder ein- bis dreifach durch Halogen, Nitro, Cyano, $C_1$-$C_4$-Alkyl, $C_1$-$C_4$-Haloalkyl, $N(C_1$-$C_3$-alkyl$)_2$, und/oder $C_1$-$C_3$-Alkoxy substituierte Phenyl- oder Benzylgruppe sein kann.

Unter dem Begriff Alkyl selbst oder als Bestandteil eines anderen Substituenten wie Alkoxy, Haloalkyl, Haloalkoxy etc., sind je nach Zahl der angegebenen Kohlenstoffatome beispielsweise die folgenden geradkettigen oder verzweigten Gruppen zu verstehen : Methyl, Ethyl, Propyl, Butyl, Pentyl, Hexyl, usw. sowie ihre Isomeren, wie z. B. Isopropyl, Isobutyl, tert.-Butyl, Isopentyl usw. Die Vorsilbe Halo in der Bezeichnung eines Substituenten bedeutet hier und im folgenden, dass dieser Substituent einfach bis perhalogeniert auftreten kann. Halogen und Halo stehen stellvertretend für Fluor, Chlor, Brom oder Jod. Haloalkyl steht somit für einen einfach bis perhalogenierten Alkylrest, wie z. B. $CHCl_2$, $CH_2F$, $CCl_3$, $CH_2Cl$, $CHF_2$, $CH_2CH_2Br$, $C_2Cl_5$, $CH_2Br$, CHBrCl usw., vorzugsweise für $CF_3$. Alkenyl steht z. B. für Allyl, Butenyl-(2) oder Butenyl-(3) sowie Ketten mit mehreren Doppelbindungen. Cycloalkyl steht je nach Zahl der genannten Kohlenstoffatome z. B. für Cyclopropyl, Cyclobutyl, Cyclopentyl, Cyclohexyl, Cycloheptyl, Cyclooctyl usw. Alkinyl bedeutet z. B. Propinyl-(2), Propargyl, Butinyl-(2) usw., vorzugsweise Propargyl.

Die Verbindungen der Formel I sind bei Raumtemperatur Oele, Harze oder überwiegend kristalline Feststoffe, die sich durch sehr wertvolle biozide Eigenschaften auszeichnen. Sie lassen sich beispielsweise auf dem Agrarsektor oder verwandten Gebieten präventiv und kurativ zur Bekämpfung phytopathogener Schädlinge, wie z. B. Pilzen einsetzen. Die erfindungsgemässen Wirkstoffe der Formel I zeichnen sich in weiten Anwendungskonzentrationen durch eine hohe biozide Aktivität und grosse Wirkungsbreite aus und können problemlos insbesondere im Agrarbereich eingesetzt werden.

Folgende Wirkstoffgruppen sind auf Grund ihrer ausgeprägten bioziden, insbesondere pflanzenfungiziden Aktivität bevorzugt :

Gruppe Ia

Verbindungen der Formel I, worin $R_1$ für Nitro, $R_2$ für Trifluormethyl, $R_3$ für Chlor stehen, $R_4$ Wasserstoff oder die Gruppe —C(O)R' bedeutet, wobei R' unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$ Wasserstoff, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$-Alkylthio, durch $N(C_1$-$C_2$-alkyl$)_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, $N(C_1$-$C_3$-alkyl$)_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind ; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder $N(C_1$-$C_4$-alkyl$)_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenylo-

xy, $C_3$-$C_4$-Alkenylthio, $C_3$-$C_4$-Alkinyloxy, $C_3$-$C_4$-Alkinylthio oder $N(C_1$-$C_3$-alkyl$)_2$ stehen.

Gruppe Ib

Verbindungen der Formel I, worin $R_1$ für Nitro, $R_2$ für Trifluormethyl, $R_3$ für Wasserstoff stehen, $R_4$ Wasserstoff oder die Gruppe —C(O)R′ bedeutet, wobei R′ unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$ Wasserstoff, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$-Alkylthio, durch $N(C_1$-$C_2$-alkyl$)_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, $N(C_1$-$C_3$-alkyl$)_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind ; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder $N(C_1$-$C_4$-alkyl$)_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenyloxy, $C_3$-$C_4$-Alkenylthio, $C_3$-$C_4$-Alkinyloxy, $C_3$-$C_4$-Alkinylthio oder $N(C_1$-$C_3$-alkyl$)_2$ stehen.

Gruppe Ic

Verbindungen der Formel I, worin $R_1$ für Trifluormethyl, $R_2$ für Nitro, $R_3$ für Wasserstoff stehen, $R_4$ Wasserstoff oder die Gruppe —C(O)R′ bedeutet, wobei R′ unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$ Wasserstoff, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$-Alkylthio, durch $N(C_1$-$C_2$-alkyl$)_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, $N(C_1$-$C_3$-alkyl$)_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind ; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder $N(C_1$-$C_4$-alkyl$)_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenyloxy, $C_3$-$C_4$-Alkenylthio, $C_3$-$C_4$-Alkinyloxy, $C_3$-$C_4$-Alkinylthio oder $N(C_1$-$C_3$-alkyl$)_2$ stehen.

Gruppe Id

Verbindungen der Formel I, worin $R_1$ für $NO_2$ steht, $R_2$ für $NO_2$ steht, $R_3$ Wasserstoff bedeutet und die übrigen Substituenten wie in der Gruppe Ia definiert sind.

Gruppe Ie

Verbindungen der Formel I, worin $R_1$ Wasserstoff bedeutet, $R_2$ für $CF_3$ oder $NO_2$ steht, $R_3$ für Wasserstoff steht und die übrigen Substituenten wie in der Gruppe Ia definiert sind.

Gruppe If

Verbindungen der Formel I, worin $R_1$ für $NO_2$ oder $CF_3$ steht, $R_2$ Wasserstoff bedeutet, $R_3$ für Wasserstoff steht und die übrigen Substituenten wie in der Gruppe Ia definiert sind.

Gruppe Ig

Verbindungen der Formel I, worin
$R_1$ für $NO_2$ oder $CF_3$ steht ;
$R_2$ für $NO_2$ oder $CF_3$ steht ;
$R_3$ Wasserstoff oder Halogen bedeutet ;
$R_4$ für Wasserstoff oder die Gruppe —C(O)R′ steht, wobei R′ unsubstituiertes oder durch Halogen, $C_1$-$C_3$-Alkoxy oder $C_1$-$C_3$-Alkylthio substituiertes $C_1$-$C_4$-Alkyl bedeutet ;
$R_5$, $R_6$ und $R_7$ unabhängig voneinander für Wasserstoff, Halogen, Nitro, Cyano, Rhodano, Mercapto, $C_1$-$C_6$-Alkyl, $C_1$-$C_6$-Haloalkyl, $C_1$-$C_6$-Alkoxy, durch Halogen, Nitro, Cyano, $C_1$-$C_3$-Alkoxy, $C_1$-$C_3$-Alkylthio und/oder $N(C_1$-$C_4$-alkyl$)_2$ substituiertes $C_1$-$C_6$-Alkoxy, $C_3$-$C_6$-Cycloalkoxy, $C_3$-$C_6$-Alkenyloxy, $C_3$-$C_6$-Alkinyloxy, $C_1$-$C_6$-Alkylthio, $C_3$-$C_6$-Alkenylthio, $C_1$-$C_6$-Alkylsulfonyl, $C_1$-$C_6$-Alkylsulfoxyl oder für die Gruppe $N(C_1$-$C_4$-alkyl$)_2$ stehen und $R_5$ zusätzlich für eine unsubstituierte oder ein- bis dreifach durch Halogen, Nitro, Cyano, $C_1$-$C_4$-Alkyl, $C_1$-$C_3$-Haloalkyl, $N(C_1$-$C_3$-alkyl$)_2$ und/oder $C_1$-$C_3$-Alkoxy substituierte Phenyl- oder Benzylgruppe sein kann.

Innerhalb der genannten Untergruppen sind insbesondere diejenigen Verbindungen bevorzugt, bei denen der Substituent $R_4$ für Wasserstoff steht.

Besonders bevorzugte Einzelsubstanzen sind z. B. :

N-(3'-Chlor-2',6'-dinitro-4'-trifluormethylphenyl)-5-amino-4-chlor-6-methoxypyrimidin (Nr. 1.1),
N-(3'-Chlor-2',6'-dinitro-4'-trifluormethylphenyl)-5-amino-4,6-dimethylthiopyrimidin (Nr. 1.2),
N-(3'-Chlor-2',6'-dinitro-4'-trifluormethylphenyl)-5-amino-4,6-ditrifluorethoxypyrimidin (Nr. 1.3),
N-(3'-Chlor-2',6'-dinitro-4'-trifluormethylphenyl)-5-amino-4,6-dichlorpyrimidin (Nr. 1.4),
N-(3'-Chlor-2',6'-dinitro-4'-trifluormethylphenyl)-5-amino-4-chlor-6-methylmercaptopyrimidin (Nr. 1.184);
N-(3'-Chlor-2',6'-dinitro-4'-trifluormethylphenyl)-5-amino-4-chlor-2-methyl-6-methoxypyrimidin (Nr. 1.205);
N-(3-Chlor-2',6'-dinitro-4'-trifluormethylphenyl)-5-amino-2-chlor-4-methoxypyrimidin (Nr. 1.51);
N-(3'-Chlor-2',6'-dinitro-4'-trifluormethylphenyl)-5-amino-2,4-dichlorpyrimidin (Nr. 1.49);
N-(3'-Chlor-2',6'-dinitro-4'-trifluormethylphenyl)-5-amino-2-chlor-4-allyloxypyrimidin (Nr. 1.210);
N(3'-Chlor-2',6'-dinitro-4'-trifluormethylphenyl)-5-amino-2-chlor-4-allylmercaptopyrimidin (Nr. 1.57);
N-(3'-Chlor-2',6'-dinitro-4'-trifluormethylphenyl)-5-amino-2-chlor-4-propargyloxypyrimidin (Nr. 1.211);
N-(3'-Chlor-2',6'-dinitro-4'-trifluormethylphenyl)-5-amino-4,6-dichlor-2-methylpyrimidin (Nr. 1.96);
N-(3'-Chlor-2',6'-dinitro-4'-trifluormethylphenyl)-5-amino-4,6-dichlor-2-phenylpyrimidin (Nr. 1.125);
N-(2',6'-Dinitro-4'-trifluormethylphenyl)-5-amino-4,6-dichlorpyrimidin (Nr. 2.4);
N-(2',4-Dinitro-6'-trifluormethylphenyl)-5-amino-4,6-dichlorpyrimidin (Nr. 3.4);
N-(2',4',6'-Trinitrophenyl)-5-amino-4,6-dichlorpyrimidin (Nr. 7.1);
N-(2'-Nitro-4'-trifluormethylphenyl)-5-amino-4,6-dichlorpyrimidin (Nr. 8.1).

Die Verbindungen der Formel I werden erfindungsgemäss dadurch hergestellt, dass man eine Verbindung der Formel II

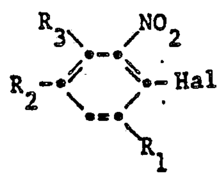

(II)

in Gegenwart einer Base mit einem Pyrimidin-Derivat der Formel III

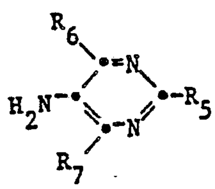

(III)

zu einer Verbindung der Formel I'

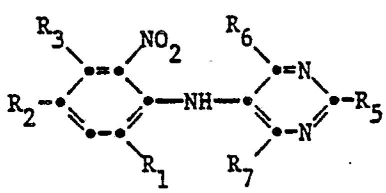

(I')

umsetzt, und letztere zur Herstellung N-acylierter Derivate mit einem reaktionsfähigen Derivat der Carbonsäure IV

$$R_4COOH \quad (IV)$$

N-acyliert, wobei die Substituenten $R_1$ bis $R_7$ die unter Formel I angegebenen Bedeutungen haben und Hal für Halogen, vorzugsweise Chlor oder Brom steht.

Für die Herstellung der Verbindungen der Formel I bzw. I' sind folgende Reaktionsbedingungen vorteilhaft:

Die N-Alkylierung von (III) mit (II) zu (I'), sowie die N-Acylierung von (I') mit (IV) zu (I) erfolgen unter Halogenwasserstoffabspaltung. Die Reaktionstemperaturen liegen bei der N-Alkylierung zwischen —60° und 150 °C, vorzugsweise —50° und + 30 °C, bei der N-Acylierung zwischen 0° und + 180 °C, vorzugsweise 0° und 150 °C bzw. am Siedepunkt des Lösungsmittels bzw. Lösungsmittelgemisches. In beiden Fällen ist die Verwendung von säurebindenden Mitteln bzw. Kondensationsmitteln vorteilhaft. Als solche kommen organische und anorganische Basen in Betracht, z. B. tertiäre Amine wie Trialkylamine

(Trimethylamin, Triethylamin, Tripropylamin usw.), Pyridin und Pyridinbasen (4-Dimethylaminopyridin, 4-Pyrrolidylaminopyridin usw.), Oxide und Hydroxide, Alkoholate, Carbonate und Hydrogencarbonate von Alkali- und Erdalkalimetallen sowie Alkaliacetate.

Die Reaktionen können in Anwesenheit von reaktionsinerten Lösungs- oder Verdünnungsmitteln durchgeführt werden. In Frage kommen beispielsweise aliphatische und aromatische Kohlenwasserstoffe wie Benzol, Toluol, Xylole, Petrolether ; halogenierte Kohlenwasserstoffe wie Chlorbenzol, Methylenchlorid, Ethylenchlorid, Chloroform, Tetrachlorkohlenstoff, Tetrachlorethylen ; Ether und etherartige Verbindungen wie Dialkylether (Diethylether, Diisopropylether, tert.-Butylmethylether usw.), Anisol, Dioxan, Tetrahydrofuran ; Nitrile wie Acetonitril, Propionitril ; N,N-dialkylierte Amide wie Dimethylformamid ; Dimethylsulfoxid ; Ketone wie Aceton, Diethylketon, Methylethylketon und Gemische solcher Lösungsmittel untereinander. In manchen Fällen kann das Acylierungs- oder Alkylierungsmittel selbst als Lösungsmittel dienen.

Die Gegenwart eines Reaktionskatalysators wie Dimethylformamid kann von Vorteil sein.

Die Reaktion (II) und (III) kann auch in einem wässrigen Zweiphasensystem nach dem allgemein bekannten Prinzip der Phasentransferkatalyse durchgeführt werden.

Für die organische, mit Wasser nicht mischbare Phase kommen dabei z. B. folgende Lösungsmittel in Frage : Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw.

Beispiele geeigneter Phasentransfer-Katalysatoren sind : Tetraalkylammoniumhalogenide, -hydrogensulfate oder -hydroxide wie Tetrabutylammoniumchlorid, -bromid, -jodid ; Triethylbenzylammoniumchlorid, -bromid ; Tetrapropylammoniumchlorid, -bromid, -jodid ; usw. Als Phasentransfer-Katalysatoren kommen auch Phosphonium-Salze in Betracht. Die Reaktionstemperaturen liegen im allgemeinen zwischen —30° und 130 °C, bzw. am Siedepunkt des Lösungsmittels oder Lösungsmittelgemisches.

Im übrigen können bei der Herstellung aller hierin genannten Ausgang-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether ; halogenierte Kohlenwasserstoffe wie Chlorbenzol, Methylenchlorid, Ethylenchlorid, Chloroform, Tetrachlorkohlenstoff, Tetrachlorethylen ; Ether und etherartige Verbindungen wie Dialkylether (Diethylether, Diisopropylether, tert.-Butylmethylether usw.), Anisol, Dioxan, Tetrahydrofuran ; Nitrile wie Acetonitril, Propionitril ; N,N-dialkylierte Amide wie Dimethylformamid ; Dimethylsulfoxid ; Ketone wie Aceton, Diethylketon, Methylethylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon, usw.

Die Ausgangsverbindungen der Formel II sind allgemein bekannt oder können nach an sich bekannten Methoden hergestellt werden. Pyrimidine der Formel III sind aus Lehrbüchern z. B. « D.J. Brown, The Pyrimidines, in Heterocyclic Compounds » bekannt oder können analog zu den dort beschriebenen Verfahren hergestellt werden.

Das beschriebene Herstellungsverfahren ist, einschliesslich aller Teilschritte, ein wichtiger Bestandteil der vorliegenden Erfindung.

In der britischen Patentschrift Nr. 1388825 werden heterocyclische Verbindungen, darunter auch Pyrimidinderivate, als Schädlingsbekämpfungsmittel, z. B. auch zur Bekämpfung phytopathogener Mikroorganismen, beschrieben.

Es wurde überraschend festgestellt, dass Verbindungen der Formel I ein für praktische Bedürfnisse sehr günstiges biozides Spektrum gegen Pilze und Bakterien, insbesondere gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen und Insekten verschont bleiben.

Als Mikrobizide sind die Wirkstoffe der Formel I z. B. gegen die den folgenden Klassen angehörenden phytopathogenen Pilze wirksam : Fungi imperfecti (z. B. Botrytis, Helminthosporium, Fusarium Septoria, Cercospora und Alternaria) ; Basidiomyceten (z. B. die Gattungen Hemileia, Rhizocotonia, Puccinia) ; insbesondere wirken sie gegen die Klasse der Ascomyceten (z. B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft somit auch Schädlingsbekämpfungsmittel, insbesondere fungizide Mittel sowie deren Verwendung auf dem Agrarsektor oder verwandten Gebieten.

Darüberhinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für den hierin offenbarten mikrobiziden Einsatz gelten im Rahmen dieser Erfindung beispielsweise folgende Pflanzenarten : Getreide ; (Weizen, Gerste, Roggen, Hafer, Reis, Sorghum und Verwandte) ; Rüben (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) ; Lorbeergewächse : (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch für den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha ; bevorzugt 100 g bis 2 kg AS/ha, insbesondere bei 200 g bis 600 g AS/ha.

Die Formulierungen d. h. die den Wirkstoff der Formel I und gegebenenfals einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z. B. Phosphatidylethanolamin, Phosphatidylserin, Phosphatidylcholin, Sphingomyelin, Phosphatidylinosit, Phosphatidylglycerin, Lysolecithin,

Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z. B. Phosphatidylcholin-Mischungen. Synthetische Phospholipide sind z. B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 g Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z. B. das Stearyltrimethylammoniumchlorid oder das Benzyldi(2-chlorethyl) ethylammoniumbromid.

Die in der Formulierungstechnik gebräuchlichen Tenside sind u. a. in folgenden Publikationen beschrieben :

« Mc Cutcheon's Detergents and Emulsifiers Annual » BC Publishing Corp., Ridgewood New Jersey, 1981 Helmut Stache « Tensid-Taschenbuch » Carl Hanser-Verlag München/Wien 1981.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbaucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet :

h = Stunde ; d = Tag ; min = Minute ; RT = Raumtemperatur ;

N = Normalität ; abs = absolut, wasserfrei, DMSO = Dimethylsulfoxid ;

DMF = Dimethylformamid. Druckangaben erfolgen in Millibar mb, oder Bar b.

Herstellungsbeispiele

Beispiel H1

Herstellung von

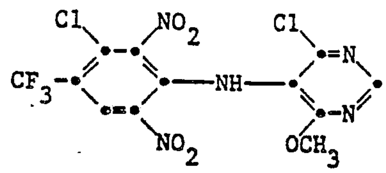

(Verb. Nr. 1.1)

N-(3'-Chlor-2',6'-dinitro-4'-trifluormethylphenyl)-5-amino-4-chlor-6-methoxypyrimidin

2,84 g (17,8 mMol) 5-Amino-4-chlor-6-methoxypyrimidin werden in 20 ml Dimethylsulfoxyd gelöst. Unter Rühren lässt man bei 15 °C gleichzeitig Lösungen von 6,24 g (20,5 mMol) 1,3-Dichlor-2,6-dinitro-4-trifluormethylbenzol und 2,30 g (20,5 mMol) K-tert.Butylat in jeweils 15 ml Dimethylsulfoxyd zutropfen. Es entsteht eine tief dunkelrote Lösung. Nach einstündigem Rühren bei 15-20 °C wird die Lösung in Eiswasser gegossen, mit Essigsäure neutralisiert und dreimal mit Chloroform extrahiert. Man trocknet die Extrakte mit Natriumsulfat und erhält nach dem Entfernen des Lösungsmittels am Rotationsverdampfer 7,9 g Rohprodukt als Oel. Zur weiteren Reinigung wird über eine Kieselgelsäule chromatographiert und man erhält 3,19 g kristallin erstarrendes Produkt, das sich aus einem Gemisch aus 2 ml Toluol und 8 ml Cyclohexan umkristallisieren lässt. Ausbeute 2,52 g gelbe Kristalle vom Smp. 114-116 °C.

Beispiel H2

Herstellung von

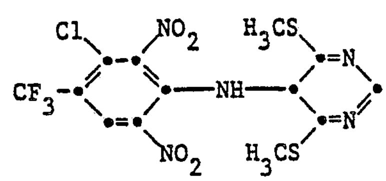

(Verb. Nr. 1.2)

N-(3'-Chlor-2',6'-dinitro-4'-trifluormethylphenyl)-5-amino-4,6-dimethylthiopyrimidin

Man löst 7,32 g (0,24 mMol) 1,3-Dichlor-2,6-dinitro-4-trifluormethylbenzol in 30 ml Dimethylsulfoxyd und lässt danach Lösungen von 3,75 g (20 mMol) 5-Amino-4,6-dimethylthiopyrimidin und 2,69 g K-tert.Butylat in jeweils 20 ml Dimethylsulfoxyd gleichzeitig bei 15 °C unter Kühlung zutropfen. Nach 15 stündigem Rühren bei 20 °C wird die Lösung in Eiswasser gegossen und nach Neutralisation mit Essigsäure dreimal mit Chloroform extrahiert. Man wascht die Extrakte mit Wasser, trocknet mit Natriumsulfat und erhält nach dem Entfernen des Chloroforms 10,7 g gelbes Oel. Die Säulenchromatographie mit Kieselgel ergibt neben 2,04 g des eingesetzten 5-Amino-4,6-di-(methylmercapto)-pyrimidins 2,8 g gelbes Produkt, das nach Umkristallisation aus 1 ml Toluol und 5 ml Cyclohexan 2,03 g der Verbindung Nr. 1.2 mit Smp. 146-7° ergibt.

Beispiel H3

Herstellung von

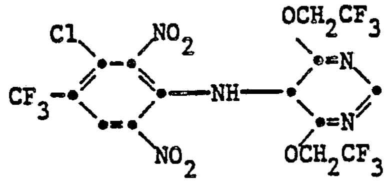

(Verb. Nr. 1.3)

N-(3'-Chlor-2',6'-dinitro-4'-trifluormethylphenyl)-5-amino-4,6-ditrifluorethoxypyrimidin

Man löst 3,36 g (11 mMol) 1,3-Dichlor-2,6-dinitro-4-trifluormethylbenzol in 20 ml Dimethylsulfoxyd und lässt sodann Lösungen von 2,62 g (9 mMol) 5-Amino-4,6-ditrifluorethoxypyrimidin und 2,47 g (22

mMol) K-tert.Butylat in jeweils 15 ml Dimethylsulfoxyd gleichzeitig unter Kühlung bei 15 °C zutropfen. Nach 3-stündigem Rühren bei Raumtemperatur enthält die dunkelrote Lösung noch Ausgangspyrimidin. Es werden weitere 2,0 g (6,9 mMol) 1,3-Dichlor-2,6-dinitro-4-trifluormethylbenzol und 1,5 g (13,4 mMol) K-tert.Butylat zugegeben ; die Aufarbeitung durch Extraktion und Säulenchromatographie ergibt 3,54 g gelbe Substanz. Durch Umkristallisation aus 1 ml Toluol und 9 ml Cyclohexan erhält man 2,00 g Produkt vom Smp. 97-99 °C.

Beispiel H4

Herstellung von

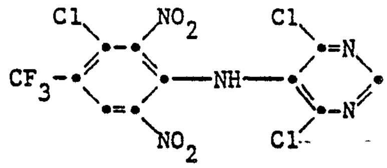

(Verb. Nr. 1.4)

N-(3'-Chlor-2',6'-dinitro-4'-trifluormethylphenyl)-5-amino-4,6-dichlorpyrimidin

Die Umsetzung von 1,64 g 5-Amino-4,6-dichlorpyrimidin mit 3,05 g 1,3-Dichlor-2,6-dinitro-4-trifluormethylbenzol in Gegenwart von 1,67 g K-tert.Butylat in DMSO analog zu Beispiel H3 ergibt 1,81 g Reinprodukt vom Smp. 127-128 °C.

Hohe Ausbeuten und reine Produkte erhält man, wenn man in den Arbeitsvorschriften H1 bis H4 statt Dimethylsulfoxyd Tetrahydrofuran als Lösungsmittel einsetzt und bei — 50° bis — 30 °C arbeitet.

Beispiel H5

Herstellung von

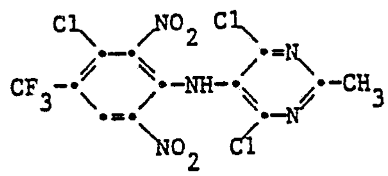

(Verb. Nr. 1.96)

N-(3'-Chlor-2',6'-dinitro-4'-trifluormethylphenyl)-5-amino 4,6-dichlor-2-methylpyrimidin

Man löst 6,71 g (0,022 Mol) 1,3-Dichlor-2,6-dinitro-4-trifluormethylbenzol mit 3,56 g (0,020 Mol) 5-Amino-4,6-dichlor-2-methylpyrimidin in 50 ml Tetrahydrofuran, kühlt auf — 30° ab, und lässt bei dieser Temperatur eine Lösung von 4,6 g (0,041 Mol) Kalium-tert.-Butylat in 50 ml Tetrahydrofuran zutropfen. Nach einer Stunde bei — 30 bis 0 °C wird das Gemisch mit Wasser und Essigester unter Zusatz von 10 ml Eisessig extrahiert. Nach dem Trocknen des Extrakts mit $Na_2SO_4$ und dem Einengen erhält man 10,5 g Rohprodukt als Oel, das mittels einer Kieselgelsäule unter Verwendung eines Gemisches aus 3 Teilen Petroläther und 1 Teil Essigester gereinigt wird. Nach dem Entfernen des Lösungsmittels am Rotationsverdampfer verbleiben 8,3 g Produkt als Rückstand. Die Umkristallisation aus einem Gemisch von Toluol und Cyclohexan ergibt 6,80 g (69,2 % der Theorie) gelbe Kristalle von Smp. 134-136 °C.

Analog zu den vorstehend beschriebenen Arbeitsweisen werden die nachfolgend aufgeführten Verbindungen der Formel I hergestellt :

(Siehe Tabellen Seite 10 ff.)

Tabelle 1 : Verbindungen der Formel

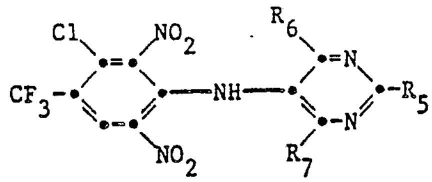

| Verb. Nr. | $R_5$ | $R_6$ | $R_7$ | physikalische Konstante [°C] |
|---|---|---|---|---|
| 1.1 | H | Cl | $OCH_3$ | Smp. 114-116 |
| 1.2 | H | $SCH_3$ | $SCH_3$ | Smp. 146-147 |
| 1.3 | H | $OCH_2CF_3$ | $OCH_2CF_3$ | Smp. 97-99 |
| 1.4 | H | Cl | Cl | Smp. 127-128 |
| 1.5 | Cl | $CH_3$ | $CH_3$ | |
| 1.6 | SCN | $CH_3$ | $CH_3$ | |
| 1.7 | $OCH_2CF_3$ | $CH_3$ | $CH_3$ | |
| 1.8 | $SCH_3$ | $CH_3$ | $CH_3$ | |
| 1.9 | $OC_2H_5$ | $CH_3$ | $CH_3$ | |
| 1.10 | Cl | $CF_3$ | $CF_3$ | |
| 1.11 | $OC_4H_9$-n | $CF_3$ | $CF_3$ | |
| 1.12 | $SCH_2CH=CH_2$ | $CF_3$ | $CF_3$ | |
| 1.13 | F | $CF_3$ | $CF_3$ | |
| 1.14 | $OCH(CH_3)_2$ | $CCl_3$ | $CCl_3$ | |
| 1.15 | Cl | H | $CH_3$ | |
| 1.16 | Br | H | $CH_3$ | |
| 1.17 | $OC_2H_5$ | H | $C_2H_5$ | |
| 1.18 | SCN | H | $CH_3$ | |
| 1.19 | Cl | H | H | Smp. 152-154 |

Tabelle 1 (Fortsetzung)

| Verb. Nr. | $R_5$ | $R_6$ | $R_7$ | physikalische Konstante [°C] |
|---|---|---|---|---|
| 1.20 | $OCH_2CH_2F$ | H | H | |
| 1.21 | $O(CH_2)_3N(C_2H_5)_2$ | H | H | |
| 1.22 | $N(CH_3)_2$ | H | H | |
| 1.23 | $O(CH_2)_2SC_2H_5$ | H | H | |
| 1.24 | Cl | Cl | Cl | |
| 1.25 | Cl | Cl | $OC_2H_5$ | |
| 1.26 | Cl | Cl | $SC_2H_5$ | |
| 1.27 | Cl | Cl | $OCH_2CF_3$ | |
| 1.28 | Cl | $OCH_3$ | $OCH_3$ | |
| 1.29 | Cl | $OCH(CF_3)_2$ | $OCH(CF_3)_2$ | |
| 1.30 | Cl | $SCH_3$ | $SCH_3$ | |
| 1.31 | Cl | Cl | $OCH_2CH_2OCH_3$ | |
| 1.32 | Cl | Cl | $OCH_2CH_2CH_2Cl$ | |
| 1.33 | $OCH_2CF_3$ | $OCH_2CF_3$ | $OCH_2CF_3$ | |
| 1.34 | F | F | F | |
| 1.35 | Cl | Cl | $OC_6H_{13}$-n | |
| 1.36 | Cl | Cl | $SC_4H_9$-n | |
| 1.37 | Cl | Cl | $OCH_2CH{=}CH_2$ | |
| 1.38 | Cl | Cl | $OCH_2C{\equiv}CH$ | |
| 1.39 | Cl | $OCH_2CH{=}CH_2$ | O-Cyclopentyl | |
| 1.40 | Br | Br | Br | |
| 1.41 | Br | $OC_2H_5$ | $SCH_2CH{=}CH_2$ | |
| 1.42 | F | $OC_4H_9$-n | $OC_4H_9$-n | |

Tabelle 1 (Fortsetzung)

| Verb. Nr. | $R_5$ | $R_6$ | $R_7$ | physikalische Konstante [°C] |
|---|---|---|---|---|
| 1.43 | Cl | $OCH_2CH_2NO_2$ | $OCH_2CH_2NO_2$ | |
| 1.44 | Cl | Cl | $O(CH_2)_3OC_3H_7$-n | |
| 1.45 | Cl | $O(CH_2)_3N(CH_3)_2$ | $O(CH_2)_3N(CH_3)_2$ | |
| 1.46 | $OC_6H_{11}$-n | $OC_6H_{11}$-n | $OC_6H_{11}$-n | |
| 1.47 | Cl | Cl | $SOCH_3$ | |
| 1.48 | Cl | Cl | $SO_2C_2H_5$ | |
| 1.49 | Cl | H | Cl | Smp. 125-126 |
| 1.50 | F | H | F | |
| 1.51 | Cl | H | $OCH_3$ | Smp. 156-157 |
| 1.52 | Cl | H | $OC_2H_5$ | |
| 1.53 | Cl | H | $OCH_2CF_3$ | |
| 1.54 | Cl | H | $OCH_2CH_2OCH_3$ | |
| 1.55 | Cl | H | $OCH(CH_3)_2$ | |
| 1.56 | Cl | H | $SC_2H_5$ | |
| 1.57 | Cl | H | $SCH_2CH{=}CH_2$ | Smp. 145-146 |
| 1.58 | Cl | H | $SO_2CH_3$ | |
| 1.59 | Cl | H | SCN | |
| 1.60 | Cl | H | $SC_4H_9$-t | |
| 1.61 | Cl | H | $O(CH_2)_3Cl$ | |
| 1.62 | F | H | $SCH_3$ | |
| 1.63 | F | H | $OCH_3$ | |
| 1.64 | Br | H | Br | |

Tabelle 1 (Fortsetzung)

| Verb. Nr. | $R_5$ | $R_6$ | $R_7$ | physikalische Konstante [°C] |
|---|---|---|---|---|
| 1.65 | Br | H | $SCH_3$ | |
| 1.66 | Cl | H | CN | |
| 1.67 | $SCH_3$ | H | $SCH_3$ | |
| 1.68 | SH | H | SH | |
| 1.69 | $OCH_3$ | H | Cl | |
| 1.70 | $SC_2H_5$ | H | Cl | |
| 1.71 | $OCH_2CF_3$ | H | Cl | |
| 1.72 | Cl | H | $O(CH_2)_3N(CH_3)_2$ | |
| 1.73 | Cl | H | $O(CH_2)_2OC_3H_7$-n | |
| 1.74 | Cl | H | $OC_6H_{11}$-n | |
| 1.75 | $OC_6H_{11}$-n | H | Cl | |
| 1.76 | $OCH_2CH=CH_2$ | H | $SCH(CH_3)_2$ | |
| 1.77 | Cl | H | $SO_2C_2H_5$ | |
| 1.78 | Cl | $CH_3$ | Cl | Smp. 154-156 |
| 1.79 | Cl | $CH_3$ | $OCH_3$ | |
| 1.80 | Cl | $CH_3$ | $SC_2H_5$ | |
| 1.81 | Cl | $CH_3$ | $OCH_2CCl_3$ | |
| 1.82 | Cl | $CH_3$ | $OCH_2C\equiv CH$ | |
| 1.83 | $SCH_3$ | $CH_3$ | $SCH_3$ | |
| 1.84 | $OCH(CH_3)_2$ | $CH_3$ | $SC_2H_5$ | |
| 1.85 | Cl | $CH_3$ | CN | |
| 1.86 | Cl | $CF_3$ | Cl | |

Tabelle 1 (Fortsetzung)

| Verb. Nr. | $R_5$ | $R_6$ | $R_7$ | physikalische Konstante [°C] |
|---|---|---|---|---|
| 1.87 | Cl | $CF_3$ | $SCH_3$ | |
| 1.88 | Cl | $CF_3$ | $OCH_3$ | |
| 1.89 | Cl | $CCl_3$ | $SC_2H_5$ | |
| 1.90 | Cl | $CCl_3$ | $OC_4H_9$-t | |
| 1.91 | Cl | $CCl_3$ | $OCH_2CF_3$ | |
| 1.92 | Cl | $CCl_3$ | $OCH_2CH_2OCH_3$ | |
| 1.93 | Cl | $CCl_3$ | SCN | |
| 1.94 | Cl | $CCl_3$ | $OC_6H_{13}$-n | |
| 1.95 | $SO_2CH_3$ | $CH_3$ | $SO_2CH_3$ | |
| 1.96 | $CH_3$ | Cl | Cl | Smp. 134-136 |
| 1.97 | $C_2H_5$ | F | F | |
| 1.98 | $C_6H_{11}$-n | Cl | $OCH_3$ | |
| 1.99 | $(CH_3)_2CH$ | Cl | $OC_2H_5$ | |
| 1.100 | $CH_3$ | Cl | $SCH_3$ | |
| 1.101 | $CH_3$ | Cl | S-$C_4H_9$-t | |
| 1.102 | $CH_3$ | Cl | $SCH_2CH=CH_2$ | |
| 1.103 | $CH_3$ | Cl | SCN | |
| 1.104 | $C_2H_5$ | Cl | O-Cyclohexyl | |
| 1.105 | $C_3H_7$-n | Cl | $O(CH_2)_3Cl$ | |
| 1.106 | $C_4H_9$-n | Cl | $O(CH_2)_2OC_2H_5$ | |
| 1.107 | $CCl_3$ | Cl | $OCH_3$ | |
| 1.108 | $CCl_3$ | Cl | $OC_2H_5$ | |

Tabelle 1 (Fortsetzung)

| Verb. Nr. | $R_5$ | $R_6$ | $R_7$ | physikalische Konstante [°C] |
|---|---|---|---|---|
| 1.109 | $CCl_3$ | Cl | $SC_2H_5$ | |
| 1.110 | $CCl_3$ | Cl | Cl | Smp. 141-143 |
| 1.111 | $CF_3$ | Cl | Cl | |
| 1.112 | $CF_3$ | Cl | $OCH_3$ | |
| 1.113 | $CF_3$ | $SCH_3$ | $SCH_3$ | |
| 1.114 | $CF_3$ | Cl | $OCH_2CF_3$ | |
| 1.115 | $CH_3$ | $OCH_3$ | $O(CH_2)_3NH_2$ | |
| 1.116 | $CH_3$ | $SC_2H_5$ | $O(CH_2)_3Cl$ | |
| 1.117 | $CH_3$ | $SCH_2CH=CH_2$ | $S-C_4H_9-n$ | |
| 1.118 | $C_2H_5$ | $OCH_2CH_2NO_2$ | $OCH(CF_3)_2$ | |
| 1.119 | $CH_3$ | Br | Br | |
| 1.120 | $CH_3$ | Br | O-Cyclopentyl | |
| 1.121 | $CH_2C_6H_5$ | Cl | Cl | |
| 1.122 | $CH_2C_6H_4Cl(4)$ | Cl | $SCH_3$ | |
| 1.123 | $CH_2C_6H_4(OCH_3)(4)$ | Cl | $OC_2H_5$ | |
| 1.124 | $CH_2C_6H_4(NO_2)(4)$ | Cl | Cl | |
| 1.125 | $C_6H_5$ | Cl | Cl | Smp. 170-171 |
| 1.126 | $C_6H_5$ | Cl | $OCH_3$ | Smp. 207-208 |
| 1.127 | $C_6H_5$ | $SCH_3$ | $SCH_3$ | Smp. 276 |
| 1.128 | $C_6H_5$ | Cl | $N(CH_3)_2$ | |
| 1.129 | $C_6H_4Cl(2)$ | Cl | Cl | |
| 1.130 | $C_6H_4Cl(2)$ | Cl | Cl | |

Tabelle 1 (Fortsetzung)

| Verb. Nr. | $R_5$ | $R_6$ | $R_7$ | physikalische Konstante [°C] |
|---|---|---|---|---|
| 1.131 | $C_6H_4Cl(3)$ | Cl | $SC_2H_5$ | |
| 1.132 | $C_6H_4Cl(3)$ | Cl | $N(C_4H_9)_2$ | |
| 1.133 | $C_6H_4Cl(3)$ | Cl | $O(CH_2)_3Cl$ | |
| 1.134 | $C_6H_4Cl(4)$ | $SCH_3$ | $SCH_3$ | |
| 1.135 | $C_6H_4Cl(4)$ | $OCH_3$ | $OC_2H_5$ | |
| 1.136 | $C_6H_4Cl(4)$ | Cl | $OC_6H_{11}$-n | |
| 1.137 | $C_6H_4CN(4)$ | Cl | $OCH_3$ | |
| 1.138 | $C_6H_4F(3)$ | Cl | Cl | |
| 1.139 | $C_6H_4F(4)$ | Cl | $SCH_3$ | |
| 1.140 | $C_6H_4(OCH_3)(2)$ | Cl | Cl | |
| 1.141 | $C_6H_4(OCH_3)(3)$ | $SC_3H_7$-n | $C_4H_9$-t | |
| 1.142 | $C_6H_4(NO_2)(4)$ | Cl | $OC_6H_{11}$-n | |
| 1.143 | $C_6H_4[N(CH_3)_2](4)$ | Cl | Cl | |
| 1.144 | $C_6H_4(CF_3)(3)$ | Cl | Cl | |
| 1.145 | $C_6H_4(CF_3)(3)$ | Cl | $OCH_3$ | |
| 1.146 | $C_6H_4(CF_3)(4)$ | Cl | $SCH_3$ | |
| 1.147 | $C_6H_3Cl_2(2,4)$ | Cl | Cl | |
| 1.148 | $C_6H_3(NO_2)_2(3,5)$ | Cl | $OC_2H_5$ | |
| 1.149 | $C_6H_3Cl_2(2,4)$ | Cl | $OC_2H_5$ | |
| 1.150 | $C_6H_3(NO_2)(3,5)$ | Cl | Cl | |
| 1.151 | $C_6H_4(CH_3)(4)$ | Cl | Cl | Smp. 159-161 |
| 1.152 | $C_6H_4(CH_3)(4)$ | Cl | $SCH_2CH{=}CH_2$ | |

Tabelle 1 (Fortsetzung)

| Verb. Nr. | $R_5$ | $R_6$ | $R_7$ | physikalische Konstante [°C] |
|---|---|---|---|---|
| 1.153 | $C_6H_4(C_4H_9\text{-t})(4)$ | Cl | Cl | |
| 1.154 | $SCH_3$ | H | $OC_3H_7\text{-n}$ | |
| 1.155 | $SCH_3$ | H | $O(CH_2)_2OCH_3$ | |
| 1.156 | $SCH_3$ | H | $O(CH_2)_3N(C_2H_5)_2$ | |
| 1.157 | $N(CH_3)_2$ | H | Cl | |
| 1.158 | $N(C_2H_5)_2$ | H | $OC_2H_5$ | |
| 1.159 | $N(CH_3)_2$ | H | $O(CH_2)_3Cl$ | |
| 1.160 | $C_6H_5$ | H | Cl | |
| 1.161 | $C_6H_5$ | H | $SC_2H_5$ | |
| 1.162 | $CH_3$ | Cl | $CH_3$ | |
| 1.163 | $CH_2C_6H_5$ | $OCH_3$ | $CH_3$ | |
| 1.164 | $C_6H_5$ | Cl | $CH_3$ | |
| 1.165 | $SCH_3$ | $OCH_2CF_3$ | $CF_3$ | |
| 1.166 | $CH_3$ | $CH_3$ | $CH_3$ | |
| 1.167 | $C_5H_{11}\text{-n}$ | $CH_3$ | $CH_3$ | |
| 1.168 | $C_6H_4(4)$ | $CF_3$ | $CF_3$ | |
| 1.169 | $C_6H_4CN(3)$ | $CF_3$ | $CF_3$ | |
| 1.170 | $N(CH_3)_2$ | $CH_3$ | $CH_3$ | |
| 1.171 | $SCH_3$ | H | $CH_3$ | |
| 1.172 | $C_2H_5$ | H | $CH_3$ | |
| 1.173 | $C_6H_4Cl(2)$ | H | $CH_3$ | |
| 1.174 | $CH_3$ | H | H | Smp. 144-145 |

Tabelle 1 (Fortsetzung)

| Verb. Nr. | $R_5$ | $R_6$ | $R_7$ | physikalische Konstante [°C] |
|---|---|---|---|---|
| 1.175 | $SCH_3$ | H | H | |
| 1.176 | $C_6H_4(CF_3)(4)$ | H | H | |
| 1.177 | $C_6H_4(OCH_3)(3)$ | H | H | |
| 1.178 | H | Cl | $OC_2H_5$ | Smp. 119-120 |
| 1.179 | H | Cl | $OCH(CH_3)C_2H_5$ | |
| 1.180 | H | Cl | O-Cyclohexyl | |
| 1.181 | H | Cl | $OCH_2CF_3$ | |
| 1.182 | H | Cl | $OCH_2CH{=}CH_2$ | Smp. 94-95 |
| 1.183 | H | Cl | $OCH_2CH_2OCH_3$ | |
| 1.184 | H | Cl | $SCH_3$ | Smp. 140-142 |
| 1.185 | H | $N(CH_3)_2$ | $SCH_3$ | |
| 1.186 | H | Cl | $SCH(CH_3)_2$ | |
| 1.187 | H | Cl | SCN | |
| 1.188 | H | SCN | SCN | |
| 1.189 | H | Cl | $S(O)CH_3$ | |
| 1.190 | H | Cl | $SO_2CH_3$ | |
| 1.191 | H | SH | SH | |
| 1.192 | H | F | F | |
| 1.193 | H | $OCH(CF_3)_2$ | $OCH(CF_3)_2$ | |
| 1.194 | H | Cl | $O(CH_2)_3Cl$ | |
| 1.195 | H | $OCH_2CH_2NO_2$ | $O(CH_2)_3OC_2H_5$ | |
| 1.196 | H | Cl | $O(CH_2)_2N(C_4H_9\text{-}n)_2$ | |
| 1.197 | H | Cl | $OCH_2CH_2NO_2$ | |
| 1.198 | H | $OCH_3$ | $O(CH_2)_3N(CH_3)_2$ | |

Tabelle 1 (Fortsetzung)

| Verb. Nr. | $R_5$ | $R_6$ | $R_7$ | physikalische Konstante [°C] |
|---|---|---|---|---|
| 1.199 | H | $SC_2H_5$ | $OCH_3$ | |
| 1.200 | H | $SCH_2CH{=}CH_2$ | $N(C_4H_9\text{-}n)_2$ | |
| 1.201 | H | $N(CH_3)_2$ | $N(C_2H_5)_2$ | |
| 1.202 | H | F | $SCH_3$ | |
| 1.203 | H | $S\text{-}C_4H_9\text{-}t$ | $O(CH_2)_2OC_4H_9\text{-}n$ | |
| 1.204 | H | Cl | $OCH_2CH_2CN$ | |
| 1.205 | $CH_3$ | Cl | $OCH_3$ | Smp. 131-132 |
| 1.206 | $OCH_3$ | $OCH_3$ | H | Smp. 144-145 |
| 1.207 | Cl | $N(C_2H_5)_2$ | H | Smp. 205 |
| 1.208 | $OCH(CH_3)_2$ | H | $OCH(CH_3)_2$ | Oel |
| 1.209 | $OCH_2CH_2OCH(CH_3)_2$ | H | $OCH_2CH_2OCH(CH_3)_2$ | Oel |
| 1.210 | Cl | $OCH_2CH{=}CH_2$ | H | Smp. 147-148 |
| 1.211 | Cl | $OCH_2C{\equiv}CH$ | H | Smp. 149-150 |
| 1.212 | H | H | H | Smp. 172-173 |
| 1.213 | $CH_3$ | Cl | $OC_2H_5$ | Smp. 164-165 |
| 1.214 | Cl | H | $SCH_3$ | Smp. 186-187 |
| 1.215 | H | $OCH_3$ | H | Smp. 120-122 |
| 1.216 | $CH_3$ | Cl | H | Smp. 115-116 |
| 1.217 | $CH_3$ | $OC_2H_5$ | H | Smp. 104-106 |
| 1.218 | H | $OCH_3$ | $OCH_3$ | Smp. 137-138 |
| 1.219 | $CH_3$ | $SCH_3$ | H | Smp. 168-170 |
| 1.220 | $C_2H_5$ | Cl | Cl | Smp. 154 |
| 1.221 | Cl | H | $OCH_2CH_2SC_2H_5$ | |
| 1.222 | Cl | H | $OCH_2CCl_2CF_3$ | |
| 1.223 | H | Cl | $OCH_2C{\equiv}CH$ | |
| 1.224 | $CH_3$ | Cl | $OCH_2CF_3$ | |
| 1.225 | $CH_3$ | Cl | $OCH_2CH{=}CH_2$ | |

Tabelle 1 (Fortsetzung)

| Verb. Nr. | $R_5$ | $R_6$ | $R_7$ | physikalische Konstante [°C] |
|---|---|---|---|---|
| 1.226 | $CH_3$ | Cl | $OCH_2C{\equiv}CH$ | |
| 1.227 | $C_2H_5$ | Cl | $OCH_3$ | |
| 1.228 | $C_2H_5$ | Cl | $SCH_3$ | |
| 1.229 | $C_2H_5$ | Cl | $OCH_2CH_2CH_3$ | |
| 1.230 | $CH(CH_3)_2$ | Cl | Cl | |
| 1.231 | $CH(CH_3)_2$ | Cl | $OCH_3$ | |
| 1.232 | $CH(CH_3)_2$ | Cl | $SCH_3$ | |
| 1.233 | $OCH_2CH{=}CH_2$ | Cl | H | |
| 1.234 | Cl | $CH_3$ | $SCH_3$ | |
| 1.235 | Cl | $CH_3$ | $OCH_2CH{=}CH_2$ | |
| 1.236 | $SCH_3$ | Cl | Cl | |
| 1.237 | $SCH_3$ | Cl | $OCH_3$ | |
| 1.238 | $SCH_3$ | Cl | $SCH_3$ | |
| 1.239 | $SCH_3$ | Cl | $OCH_2CF_3$ | |
| 1.240 | $SCH_3$ | Cl | $OCH_2CH{=}CH_2$ | |

Tabelle 2 : Verbindungen der Formel

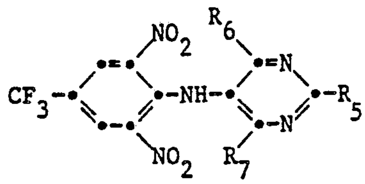

| Verb. Nr. | $R_5$ | $R_6$ | $R_7$ | physikalische Konstante [°C] |
|---|---|---|---|---|
| 2.1 | H | Cl | $OCH_3$ | Smp. 134-135 |
| 2.2 | H | $SCH_3$ | $SCH_3$ | |
| 2.3 | H | $OCH_2CF_3$ | $OCH_2CF_3$ | |
| 2.4 | H | Cl | Cl | Smp. 159-160 |
| 2.5 | Cl | $CH_3$ | $CH_3$ | |
| 2.6 | SCN | $CH_3$ | $CH_3$ | |
| 2.7 | $OCH_2CF_3$ | $CH_3$ | $CH_3$ | |
| 2.8 | $SCH_3$ | $CH_3$ | $CH_3$ | |
| 2.9 | $OC_2H_5$ | $CH_3$ | $CH_3$ | |
| 2.10 | Cl | $CF_3$ | $CF_3$ | |
| 2.11 | $OC_4H_9$-n | $CF_3$ | $CF_3$ | |
| 2.12 | $SCH_2CH=CH_2$ | $CF_3$ | $CF_3$ | |
| 2.13 | F | $CF_3$ | $CF_3$ | |
| 2.14 | $OCH(CH_3)_2$ | $CCl_3$ | $CCl_3$ | |
| 2.15 | Cl | H | $CH_3$ | |
| 2.16 | Br | H | $CH_3$ | |
| 2.17 | $OC_2H_5$ | H | $C_2H_5$ | |
| 2.18 | SCN | H | $CH_3$ | |
| 2.19 | Cl | H | H | |

Tabelle 2 (Fortsetzung)

| Verb. Nr. | $R_5$ | $R_6$ | $R_7$ | physikalische Konstante [°C] |
|---|---|---|---|---|
| 2.20 | $OCH_2CH_2F$ | H | H | |
| 2.21 | $O(CH_2)_3N(C_2H_5)_2$ | H | H | |
| 2.22 | $N(CH_3)_2$ | H | H | |
| 2.23 | $O(CH_2)_2SC_2H_5$ | H | H | |
| 2.24 | Cl | Cl | Cl | |
| 2.25 | Cl | Cl | $OC_2H_5$ | |
| 2.26 | Cl | Cl | $SC_2H_5$ | |
| 2.27 | Cl | Cl | $OCH_2CF_3$ | |
| 2.28 | Cl | $OCH_3$ | $OCH_3$ | |
| 2.29 | Cl | $OCH(CF_3)_2$ | $OCH(CF_3)_2$ | |
| 2.30 | Cl | $SCH_3$ | $SCH_3$ | |
| 2.31 | Cl | Cl | $OCH_2CH_2OCH_3$ | |
| 2.32 | Cl | Cl | $OCH_2CH_2CH_2Cl$ | |
| 2.33 | $OCH_2CF_3$ | $OCH_2CF_3$ | $OCH_2CF_3$ | |
| 2.34 | F | F | F | |
| 2.35 | Cl | Cl | $OC_6H_{13}$-n | |
| 2.36 | Cl | Cl | $SC_4H_9$-n | |
| 2.37 | Cl | Cl | $OCH_2CH{=}CH_2$ | |
| 2.38 | Cl | Cl | $OCH_2C{\equiv}CH$ | |
| 2.39 | Cl | $OCH_2CH{=}CH_2$ | O-Cyclopentyl | |
| 2.40 | Br | Br | Br | |
| 2.41 | Br | $OC_2H_5$ | $SCH_2CH{=}CH_2$ | |
| 2.42 | F | $OC_4H_9$-n | $OC_4H_9$-n | |

Tabelle 2 (Fortsetzung)

| Verb. Nr. | $R_5$ | $R_6$ | $R_7$ | physikalische Konstante [°C] |
|---|---|---|---|---|
| 2.43 | Cl | $OCH_2CH_2NO_2$ | $OCH_2CH_2NO_2$ | |
| 2.44 | Cl | Cl | $O(CH_2)_3OC_3H_7$-n | |
| 2.45 | Cl | $O(CH_2)_3N(CH_3)_2$ | $O(CH_2)_3N(CH_3)_2$ | |
| 2.46 | $OC_6H_{11}$-n | $OC_6H_{11}$-n | $OC_6H_{11}$-n | |
| 2.47 | Cl | Cl | $SOCH_3$ | |
| 2.48 | Cl | Cl | $SO_2C_2H_5$ | |
| 2.49 | Cl | H | Cl | Smp. 116-117 |
| 2.50 | F | H | F | |
| 2.51 | Cl | H | $OCH_3$ | Smp. 145-146 |
| 2.52 | Cl | H | $OC_2H_5$ | |
| 2.53 | Cl | H | $OCH_2CF_3$ | |
| 2.54 | Cl | H | $OCH_2CH_2OCH_3$ | |
| 2.55 | Cl | H | $OCH(CH_3)_2$ | |
| 2.56 | Cl | H | $SC_2H_5$ | |
| 2.57 | Cl | H | $SCH_2CH=CH_2$ | |
| 2.58 | Cl | H | $SO_2CH_3$ | |
| 2.59 | Cl | H | SCN | |
| 2.60 | Cl | H | $SC_4H_9$-t | |
| 2.61 | Cl | H | $O(CH_2)_3Cl$ | |
| 2.62 | F | H | $SCH_3$ | |
| 2.63 | F | H | $OCH_3$ | |
| 2.64 | Br | H | Br | |

Tabelle 2 (Fortsetzung)

| Verb. Nr. | $R_5$ | $R_6$ | $R_7$ | physikalische Konstante [°C] |
|---|---|---|---|---|
| 2.65 | Br | H | $SCH_3$ | |
| 2.66 | Cl | H | CN | |
| 2.67 | $SCH_3$ | H | $SCH_3$ | |
| 2.68 | SH | H | SH | |
| 2.69 | $OCH_3$ | H | Cl | |
| 2.70 | $SC_2H_5$ | H | Cl | |
| 2.71 | $OCH_2CF_3$ | H | Cl | |
| 2.72 | Cl | H | $O(CH_2)_3N(CH_3)_2$ | |
| 2.73 | Cl | H | $O(CH_2)_2OC_3H_7$-n | |
| 2.74 | Cl | H | $OC_6H_{11}$-n | |
| 2.75 | $OC_6H_{11}$-n | H | Cl | |
| 2.76 | $OCH_2CH{=}CH_2$ | H | $SCH(CH_3)_2$ | |
| 2.77 | Cl | H | $SO_2C_2H_5$ | |
| 2.78 | Cl | $CH_3$ | Cl | |
| 2.79 | Cl | $CH_3$ | $OCH_3$ | |
| 2.80 | Cl | $CH_3$ | $SC_2H_5$ | |
| 2.81 | Cl | $CH_3$ | $OCH_2CCl_3$ | |
| 2.82 | Cl | $CH_3$ | $OCH_2C{\equiv}CH$ | |
| 2.83 | $SCH_3$ | $CH_3$ | $SCH_3$ | |
| 2.84 | $OCH(CH_3)_2$ | $CH_3$ | $SC_2H_5$ | |
| 2.85 | Cl | $CH_3$ | CN | |
| 2.86 | Cl | $CF_3$ | Cl | |

Tabelle 2 (Fortsetzung)

| Verb. Nr. | $R_5$ | $R_6$ | $R_7$ | physikalische Konstante [°C] |
|---|---|---|---|---|
| 2.87 | Cl | $CF_3$ | $SCH_3$ | |
| 2.88 | Cl | $CF_3$ | $OCH_3$ | |
| 2.89 | Cl | $CCl_3$ | $SC_2H_5$ | |
| 2.90 | Cl | $CCl_3$ | $OC_4H_9$-t | |
| 2.91 | Cl | $CCl_3$ | $OCH_2CF_3$ | |
| 2.92 | Cl | $CCl_3$ | $OCH_2CH_2OCH_3$ | |
| 2.93 | Cl | $CCl_3$ | SCN | |
| 2.94 | Cl | $CCl_3$ | $OC_6H_{13}$-n | |
| 2.95 | $SO_2CH_3$ | $CH_3$ | $SO_2CH_3$ | |
| 2.96 | $CH_3$ | Cl | Cl | Smp. 143-144 |
| 2.97 | $C_2H_5$ | F | F | |
| 2.98 | $C_6H_{11}$-n | Cl | $OCH_3$ | |
| 2.99 | $(CH_3)_2CH$ | Cl | $OC_2H_5$ | |
| 2.100 | $CH_3$ | Cl | $SCH_3$ | |
| 2.101 | $CH_3$ | Cl | S-$C_4H_9$-t | |
| 2.102 | $CH_3$ | Cl | $SCH_2CH=CH_2$ | |
| 2.103 | $CH_3$ | Cl | SCN | |
| 2.104 | $C_2H_5$ | Cl | O-Cyclohexyl | |
| 2.105 | $C_3H_7$-n | Cl | $O(CH_2)_3Cl$ | |
| 2.106 | $C_4H_9$-n | Cl | $O(CH_2)_2OC_2H_5$ | |
| 2.107 | $CCl_3$ | Cl | $OCH_3$ | |
| 2.108 | $CCl_3$ | Cl | $OC_2H_5$ | |

Tabelle 2 (Fortsetzung)

| Verb. Nr. | $R_5$ | $R_6$ | $R_7$ | physikalische Konstante [°C] |
|---|---|---|---|---|
| 2.109 | $CCl_3$ | Cl | $SC_2H_5$ | |
| 2.110 | $CCl_3$ | Cl | Cl | |
| 2.111 | $CF_3$ | Cl | Cl | |
| 2.112 | $CF_3$ | Cl | $OCH_3$ | |
| 2.113 | $CF_3$ | $SCH_3$ | $SCH_3$ | |
| 2.114 | $CF_3$ | Cl | $OCH_2CF_3$ | |
| 2.115 | $CH_3$ | $OCH_3$ | $O(CH_2)_3NH_2$ | |
| 2.116 | $CH_3$ | $SC_2H_5$ | $O(CH_2)_3Cl$ | |
| 2.117 | $CH_3$ | $SCH_2CH{=}CH_2$ | $S{-}C_4H_9{-}n$ | |
| 2.118 | $C_2H_5$ | $OCH_2CH_2NO_2$ | $OCH(CF_3)_2$ | |
| 2.119 | $CH_3$ | Br | Br | |
| 2.120 | $CH_3$ | Br | O-Cyclo-pentyl | |
| 2.121 | $CH_2C_6H_5$ | Cl | Cl | |
| 2.122 | $CH_2C_6H_4Cl(4)$ | Cl | $SCH_3$ | |
| 2.123 | $CH_2C_6H_4(OCH_3)(4)$ | Cl | $OC_2H_5$ | |
| 2.124 | $CH_2C_6H_4(NO_2)(4)$ | Cl | Cl | |
| 2.125 | $C_6H_5$ | Cl | Cl | |
| 2.126 | $C_6H_5$ | Cl | $OCH_3$ | |
| 2.127 | $C_6H_5$ | Cl | $SCH_3$ | |
| 2.128 | $C_6H_5$ | Cl | $N(CH_3)_2$ | |
| 2.129 | $C_6H_4Cl(2)$ | Cl | Cl | |
| 2.130 | $C_6H_4Cl(2)$ | Cl | Cl | |

Tabelle 2 (Fortsetzung)

| Verb. Nr. | $R_5$ | $R_6$ | $R_7$ | physikalische Konstante [°C] |
|---|---|---|---|---|
| 2.131 | $C_6H_4Cl(3)$ | Cl | $SC_2H_5$ | |
| 2.132 | $C_6H_4Cl(3)$ | Cl | $N(C_4H_9)_2$ | |
| 2.133 | $C_6H_4Cl(3)$ | Cl | $O(CH_2)_3Cl$ | |
| 2.134 | $C_6H_4Cl(4)$ | $SCH_3$ | $SCH_3$ | |
| 2.135 | $C_6H_4Cl(4)$ | $OCH_3$ | $OC_2H_5$ | |
| 2.136 | $C_6H_4Cl(4)$ | Cl | $OC_6H_{11}$-n | |
| 2.137 | $C_6H_4CN(4)$ | Cl | $OCH_3$ | |
| 2.138 | $C_6H_4F(3)$ | Cl | Cl | |
| 2.139 | $C_6H_4F(4)$ | Cl | $SCH_3$ | |
| 2.140 | $C_6H_4(OCH_3)(2)$ | Cl | Cl | |
| 2.141 | $C_6H_4(OCH_3)(3)$ | $SC_3H_7$-n | $C_4H_9$-t | |
| 2.142 | $C_6H_4(NO_2)(4)$ | Cl | $OC_6H_{11}$-n | |
| 2.143 | $C_6H_4[N(CH_3)_2](4)$ | Cl | Cl | |
| 2.144 | $C_6H_4(CF_3)(3)$ | Cl | Cl | |
| 2.145 | $C_6H_4(CF_3)(3)$ | Cl | $OCH_3$ | |
| 2.146 | $C_6H_4(CF_3)(4)$ | Cl | $SCH_3$ | |
| 2.147 | $C_6H_3Cl_2(2,4)$ | Cl | Cl | |
| 2.148 | $C_6H_3(NO_2)_2(3,5)$ | Cl | $OC_2H_5$ | |
| 2.149 | $C_6H_3Cl_2(2,4)$ | Cl | $OC_2H_5$ | |
| 2.150 | $C_6H_3(NO_2)(3,5)$ | Cl | Cl | |
| 2.151 | $C_6H_4(CH_3)(4)$ | Cl | Cl | |
| 2.152 | $C_6H_4(CH_3)(4)$ | Cl | $SCH_2CH{=}CH_2$ | |

Tabelle 2 (Fortsetzung)

| Verb. Nr. | $R_5$ | $R_6$ | $R_7$ | physikalische Konstante [°C] |
|---|---|---|---|---|
| 2.153 | $C_6H_4(C_4H_9$-t)(4) | Cl | Cl | |
| 2.154 | $SCH_3$ | H | $OC_3H_7$-n | |
| 2.155 | $SCH_3$ | H | $O(CH_2)_2OCH_3$ | |
| 2.156 | $SCH_3$ | H | $O(CH_2)_3N(C_2H_5)_2$ | |
| 2.157 | $N(CH_3)_2$ | H | Cl | |
| 2.158 | $N(C_2H_5)_2$ | H | $OC_2H_5$ | |
| 2.159 | $N(CH_3)_2$ | H | $O(CH_2)_3Cl$ | |
| 2.160 | $C_6H_5$ | H | Cl | |
| 2.161 | $C_6H_5$ | H | $SC_2H_5$ | |
| 2.162 | $CH_3$ | Cl | $CH_3$ | |
| 2.163 | $CH_2C_6H_5$ | $OCH_3$ | $CH_3$ | |
| 2.164 | $C_6H_5$ | Cl | $CH_3$ | |
| 2.165 | $SCH_3$ | $OCH_2CF_3$ | $CF_3$ | |
| 2.166 | $CH_3$ | $CH_3$ | $CH_3$ | |
| 2.167 | $C_5H_{11}$-n | $CH_3$ | $CH_3$ | |
| 2.168 | $C_6H_4$(4) | $CF_3$ | $CF_3$ | |
| 2.169 | $C_6H_4CN(3)$ | $CF_3$ | $CF_3$ | |
| 2.170 | $N(CH_3)_2$ | $CH_3$ | $CH_3$ | |
| 2.171 | $SCH_3$ | H | $CH_3$ | |
| 2.172 | $C_2H_5$ | H | $CH_3$ | |
| 2.173 | $C_6H_4Cl(2)$ | H | $CH_3$ | |
| 2.174 | $CH_3$ | H | H | Smp. 168-169 |

Tabelle 2 (Fortsetzung)

| Verb. Nr. | $R_5$ | $R_6$ | $R_7$ | physikalische Konstante [°C] |
|---|---|---|---|---|
| 2.175 | $SCH_3$ | H | H | |
| 2.176 | $C_6H_4(CF_3)(4)$ | H | H | |
| 2.177 | $C_6H_4(OCH_3)(3)$ | H | H | |
| 2.178 | H | Cl | $OC_2H_5$ | |
| 2.179 | H | Cl | $OCH(CH_3)C_2H_5$ | |
| 2.180 | H | Cl | O-Cyclohexyl | |
| 2.181 | H | Cl | $OCH_2CF_3$ | |
| 2.182 | H | Cl | $OCH_2CH=CH_2$ | Smp. 71-73 |
| 2.183 | H | Cl | $OCH_2CH_2OCH_3$ | |
| 2.184 | H | Cl | $SCH_3$ | |
| 2.185 | H | Cl | $SCH_3$ | |
| 2.186 | H | Cl | $SCH(CH_3)_2$ | |
| 2.187 | H | Cl | SCN | |
| 2.188 | H | SCN | SCN | |
| 2.189 | H | Cl | $S(O)CH_3$ | |
| 2.190 | H | Cl | $SO_2CH_3$ | |
| 2.191 | H | SH | SH | |
| 2.192 | H | F | F | |
| 2.193 | H | $OCH(CF_3)_2$ | $OCH(CF_3)_2$ | |
| 2.194 | H | Cl | $O(CH_2)_3Cl$ | |
| 2.195 | H | $OCH_2CH_2NO_2$ | $O(CH_2)_3OC_2H_5$ | |
| 2.196 | H | Cl | $O(CH_2)_2N(C_4H_9\text{-}n)_2$ | |
| 2.197 | H | Cl | $OCH_2CH_2NO_2$ | |
| 2.198 | H | $OCH_3$ | $O(CH_2)_3N(CH_3)_2$ | |

Tabelle 2 (Fortsetzung)

| Verb. Nr. | $R_5$ | $R_6$ | $R_7$ | physikalische Konstante [°C] |
|---|---|---|---|---|
| 2.199 | H | $SC_2H_5$ | $OCH_3$ | |
| 2.200 | H | $SCH_2CH{=}CH_2$ | $N(C_4H_9\text{-}n)_2$ | |
| 2.201 | H | $N(CH_3)_2$ | $N(C_2H_5)_2$ | |
| 2.202 | H | F | $SCH_3$ | |
| 2.203 | H | $S\text{-}C_4H_9\text{-}t$ | $O(CH_2)_2OC_4H_9\text{-}n$ | |
| 2.204 | H | Cl | $OCH_2CH_2CN$ | |
| 2.205 | $OCH(CH_3)_2$ | $OCH(CH_3)_2$ | H | Oel |
| 2.206 | $OCH_2CH_2OCH(CH_3)_2$ | H | $OCH_2CH_2OCH(CH_3)_2$ | Oel |
| 2.207 | Cl | H | $OCH_2CH{=}CH_2$ | Smp. 74-75 |
| 2.208 | Cl | H | $OCH_2C{\equiv}CH$ | Smp. 91-92 |
| 2.209 | H | H | H | Smp. 134-135 |
| 2.210 | $CH_3$ | Cl | $OC_2H_5$ | Smp. 110-111 |
| 2.211 | $CH_3$ | $OCH_3$ | Cl | Smp. 143-144 |
| 2.212 | $C_2H_5$ | Cl | Cl | Smp. 90-91 |
| 2.213 | Cl | H | $OCH_2CH_2SC_2H_5$ | |
| 2.214 | Cl | H | $OCH_2CCl_2CF_3$ | |
| 2.215 | H | Cl | $OCH_2C{\equiv}CH$ | |
| 2.216 | $CH_3$ | Cl | $OCH_2CF_3$ | |
| 2.217 | $CH_3$ | Cl | $OCH_2CH{=}CH_2$ | |
| 2.218 | $CH_3$ | Cl | $OCH_2C{\equiv}CH$ | |
| 2.219 | $C_2H_5$ | Cl | $OCH_3$ | |

Tabelle 2 (Fortsetzung)

| Verb. Nr. | $R_5$ | $R_6$ | $R_7$ | physikalische Konstante [°C] |
|---|---|---|---|---|
| 2.220 | $C_2H_5$ | Cl | $SCH_3$ | |
| 2.221 | $C_2H_5$ | Cl | $OCH_2CH_2CH_3$ | |
| 2.222 | $CH(CH_3)_2$ | Cl | Cl | |
| 2.223 | $CH(CH_3)_2$ | Cl | $OCH_3$ | |
| 2.224 | $CH(CH_3)_2$ | Cl | $SCH_3$ | |
| 2.225 | $OCH_2CH{=}CH_2$ | Cl | H | |
| 2.226 | Cl | $CH_3$ | $SCH_3$ | |
| 2.227 | Cl | $CH_3$ | $OCH_2CH{=}CH_2$ | |
| 2.228 | $SCH_3$ | Cl | Cl | |
| 2.229 | $SCH_3$ | Cl | $OCH_3$ | |
| 2.230 | $SCH_3$ | Cl | $SCH_3$ | |
| 2.231 | $SCH_3$ | Cl | $OCH_2CF_3$ | |
| 2.232 | $SCH_3$ | Cl | $OCH_2CH{=}CH_2$ | |

Tabelle 3 : Verbindungen der Formel

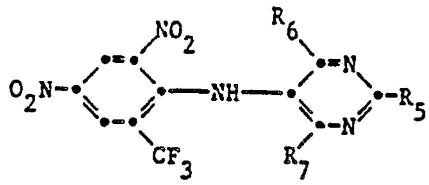

| Verb. Nr. | $R_5$ | $R_6$ | $R_7$ | physikalische Konstante [°C] |
|---|---|---|---|---|
| 3.1 | H | Cl | $OCH_3$ | Smp. 75-77 |
| 3.2 | H | $SCH_3$ | $SCH_3$ | |
| 3.3 | H | $OCH_2CF_3$ | $OCH_2CF_3$ | |
| 3.4 | H | Cl | Cl | Smp. 153-154 |
| 3.5 | Cl | $CH_3$ | $CH_3$ | |
| 3.6 | SCN | $CH_3$ | $CH_3$ | |
| 3.7 | $OCH_2CF_3$ | $CH_3$ | $CH_3$ | |
| 3.8 | $SCH_3$ | $CH_3$ | $CH_3$ | |
| 3.9 | $OC_2H_5$ | $CH_3$ | $CH_3$ | |
| 3.10 | Cl | $CF_3$ | $CF_3$ | |
| 3.11 | $OC_4H_9$-n | $CF_3$ | $CF_3$ | |
| 3.12 | $SCH_2CH=CH_2$ | $CF_3$ | $CF_3$ | |
| 3.13 | F | $CF_3$ | $CF_3$ | |
| 3.14 | $OCH(CH_3)_2$ | $CCl_3$ | $CCl_3$ | |
| 3.15 | Cl | H | $CH_3$ | |
| 3.16 | Br | H | $CH_3$ | |
| 3.17 | $OC_2H_5$ | H | $C_2H_5$ | |
| 3.18 | SCN | H | $CH_3$ | |
| 3.19 | Cl | H | H | |

Tabelle 3 (Fortsetzung)

| Verb. Nr. | $R_5$ | $R_6$ | $R_7$ | physikalische Konstante [°C] |
|---|---|---|---|---|
| 3.20 | $OCH_2CH_2F$ | H | H | |
| 3.21 | $O(CH_2)_3N(C_2H_5)_2$ | H | H | |
| 3.22 | $N(CH_3)_2$ | H | H | |
| 3.23 | $O(CH_2)_2SC_2H_5$ | H | H | |
| 3.24 | Cl | Cl | Cl | |
| 3.25 | Cl | Cl | $OC_2H_5$ | |
| 3.26 | Cl | Cl | $SC_2H_5$ | |
| 3.27 | Cl | Cl | $OCH_2CF_3$ | |
| 3.28 | Cl | $OCH_3$ | $OCH_3$ | |
| 3.29 | Cl | $OCH(CF_3)_2$ | $OCH(CF_3)_2$ | |
| 3.30 | Cl | $SCH_3$ | $SCH_3$ | |
| 3.31 | Cl | Cl | $OCH_2CH_2OCH_3$ | |
| 3.32 | Cl | Cl | $OCH_2CH_2CH_2Cl$ | |
| 3.33 | $OCH_2CF_3$ | $OCH_2CF_3$ | $OCH_2CF_3$ | |
| 3.34 | F | F | F | |
| 3.35 | Cl | Cl | $OC_6H_{13}$-n | |
| 3.36 | Cl | Cl | $SC_4H_9$-n | |
| 3.37 | Cl | Cl | $OCH_2CH{=}CH_2$ | |
| 3.38 | Cl | Cl | $OCH_2C{\equiv}CH$ | |
| 3.39 | Cl | $OCH_2CH{=}CH_2$ | O-Cyclopentyl | |
| 3.40 | Br | Br | Br | |
| 3.41 | Br | $OC_2H_5$ | $SCH_2CH{=}CH_2$ | |
| 3.42 | F | $OC_4H_9$-n | $OC_4H_9$-n | |

Tabelle 3 (Fortsetzung)

| Verb. Nr. | $R_5$ | $R_6$ | $R_7$ | physikalische Konstante [°C] |
|---|---|---|---|---|
| 3.43 | Cl | $OCH_2CH_2NO_2$ | $OCH_2CH_2NO_2$ | |
| 3.44 | Cl | Cl | $O(CH_2)_3OC_3H_7$-n | |
| 3.45 | Cl | $O(CH_2)_3N(CH_3)_2$ | $O(CH_2)_3N(CH_3)_2$ | |
| 3.46 | $OC_6H_{11}$-n | $OC_6H_{11}$-n | $OC_6H_{11}$-n | |
| 3.47 | Cl | Cl | $SOCH_3$ | |
| 3.48 | Cl | Cl | $SO_2C_2H_5$ | |
| 3.49 | Cl | H | Cl | Oel |
| 3.50 | F | H | F | |
| 3.51 | Cl | H | $OCH_3$ | Oel |
| 3.52 | Cl | H | $OC_2H_5$ | |
| 3.53 | Cl | H | $OCH_2CF_3$ | |
| 3.54 | Cl | H | $OCH_2CH_2OCH_3$ | |
| 3.55 | Cl | H | $OCH(CH_3)_2$ | |
| 3.56 | Cl | H | $SC_2H_5$ | |
| 3.57 | Cl | H | $SCH_2CH=CH_2$ | |
| 3.58 | Cl | H | $SO_2CH_3$ | |
| 3.59 | Cl | H | SCN | |
| 3.60 | Cl | H | $SC_4H_9$-t | |
| 3.61 | Cl | H | $O(CH_2)_3Cl$ | |
| 3.62 | F | H | $SCH_3$ | |
| 3.63 | F | H | $OCH_3$ | |
| 3.64 | Br | H | Br | |

Tabelle 3 (Fortsetzung)

| Verb. Nr. | $R_5$ | $R_6$ | $R_7$ | physikalische Konstante [°C] |
|---|---|---|---|---|
| 3.65 | Br | H | $SCH_3$ | |
| 3.66 | Cl | H | CN | |
| 3.67 | $SCH_3$ | H | $SCH_3$ | |
| 3.68 | SH | H | SH | |
| 3.69 | $OCH_3$ | H | Cl | |
| 3.70 | $SC_2H_5$ | H | Cl | |
| 3.71 | $OCH_2CF_3$ | H | Cl | |
| 3.72 | Cl | H | $O(CH_2)_3N(CH_3)_2$ | |
| 3.73 | Cl | H | $O(CH_2)_2OC_3H_7$-n | |
| 3.74 | Cl | H | $OC_6H_{11}$-n | |
| 3.75 | $OC_6H_{11}$-n | H | Cl | |
| 3.76 | $OCH_2CH=CH_2$ | H | $SCH(CH_3)_2$ | |
| 3.77 | Cl | H | $SO_2C_2H_5$ | |
| 3.78 | Cl | $CH_3$ | Cl | |
| 3.79 | Cl | $CH_3$ | $OCH_3$ | |
| 3.80 | Cl | $CH_3$ | $SC_2H_5$ | |
| 3.81 | Cl | $CH_3$ | $OCH_2CCl_3$ | |
| 3.82 | Cl | $CH_3$ | $OCH_2C{\equiv}CH$ | |
| 3.83 | $SCH_3$ | $CH_3$ | $SCH_3$ | |
| 3.84 | $OCH(CH_3)_2$ | $CH_3$ | $SC_2H_5$ | |
| 3.85 | Cl | $CH_3$ | CN | |
| 3.86 | Cl | $CF_3$ | Cl | |

Tabelle 3 (Fortsetzung)

| Verb. Nr. | $R_5$ | $R_6$ | $R_7$ | physikalische Konstante [°C] |
|---|---|---|---|---|
| 3.87 | Cl | $CF_3$ | $SCH_3$ | |
| 3.88 | Cl | $CF_3$ | $OCH_3$ | |
| 3.89 | Cl | $CCl_3$ | $SC_2H_5$ | |
| 3.90 | Cl | $CCl_3$ | $OC_4H_9$-t | |
| 3.91 | Cl | $CCl_3$ | $OCH_2CF_3$ | |
| 3.92 | Cl | $CCl_3$ | $OCH_2CH_2OCH_3$ | |
| 3.93 | Cl | $CCl_3$ | SCN | |
| 3.94 | Cl | $CCl_3$ | $OC_6H_{13}$-n | |
| 3.95 | $SO_2CH_3$ | $CH_3$ | $SO_2CH_3$ | |
| 3.96 | $CH_3$ | Cl | Cl | |
| 3.97 | $C_2H_5$ | F | F | |
| 3.98 | $C_6H_{11}$-n | Cl | $OCH_3$ | |
| 3.99 | $(CH_3)_2CH$ | Cl | $OC_2H_5$ | |
| 3.100 | $CH_3$ | Cl | $SCH_3$ | |
| 3.101 | $CH_3$ | Cl | $S-C_4H_9$-t | |
| 3.102 | $CH_3$ | Cl | $SCH_2CH=CH_2$ | |
| 3.103 | $CH_3$ | Cl | SCN | |
| 3.104 | $C_2H_5$ | Cl | O-Cyclohexyl | |
| 3.105 | $C_3H_7$-n | Cl | $O(CH_2)_3Cl$ | |
| 3.106 | $C_4H_9$-n | Cl | $O(CH_2)_2OC_2H_5$ | |
| 3.107 | $CCl_3$ | Cl | $OCH_3$ | |
| 3.108 | $CCl_3$ | Cl | $OC_2H_5$ | |

Tabelle 3 (Fortsetzung)

| Verb. Nr. | $R_5$ | $R_6$ | $R_7$ | physikalische Konstante [°C] |
|---|---|---|---|---|
| 3.109 | $CCl_3$ | Cl | $SC_2H_5$ | |
| 3.110 | $CCl_3$ | Cl | Cl | |
| 3.111 | $CF_3$ | Cl | Cl | |
| 3.112 | $CF_3$ | Cl | $OCH_3$ | |
| 3.113 | $CF_3$ | $SCH_3$ | $SCH_3$ | |
| 3.114 | $CF_3$ | Cl | $OCH_2CF_3$ | |
| 3.115 | $CH_3$ | $OCH_3$ | $O(CH_2)_3NH_2$ | |
| 3.116 | $CH_3$ | $SC_2H_5$ | $O(CH_2)_3Cl$ | |
| 3.117 | $CH_3$ | $SCH_2CH=CH_2$ | $S-C_4H_9-n$ | |
| 3.118 | $C_2H_5$ | $OCH_2CH_2NO_2$ | $OCH(CF_3)_2$ | |
| 3.119 | $CH_3$ | Br | Br | |
| 3.120 | $CH_3$ | Br | O-Cyclo-pentyl | |
| 3.121 | $CH_2C_6H_5$ | Cl | Cl | |
| 3.122 | $CH_2C_6H_4Cl(4)$ | Cl | $SCH_3$ | |
| 3.123 | $CH_2C_6H_4(OCH_3)(4)$ | Cl | $OC_2H_5$ | |
| 3.124 | $CH_2C_6H_4(NO_2)(4)$ | Cl | Cl | |
| 3.125 | $C_6H_5$ | Cl | Cl | |
| 3.126 | $C_6H_5$ | Cl | $OCH_3$ | |
| 3.127 | $C_6H_5$ | Cl | $SCH_3$ | |
| 3.128 | $C_6H_5$ | Cl | $N(CH_3)_2$ | |
| 3.129 | $C_6H_4Cl(2)$ | Cl | Cl | |
| 3.130 | $C_6H_4Cl(2)$ | Cl | Cl | |

Tabelle 3 (Fortsetzung)

| Verb. Nr. | $R_5$ | $R_6$ | $R_7$ | physikalische Konstante [°C] |
|---|---|---|---|---|
| 3.131 | $C_6H_4Cl(3)$ | Cl | $SC_2H_5$ | |
| 3.132 | $C_6H_4Cl(3)$ | Cl | $N(C_4H_9)_2$ | |
| 3.133 | $C_6H_4Cl(3)$ | Cl | $O(CH_2)_3Cl$ | |
| 3.134 | $C_6H_4Cl(4)$ | $SCH_3$ | $SCH_3$ | |
| 3.135 | $C_6H_4Cl(4)$ | $OCH_3$ | $OC_2H_5$ | |
| 3.136 | $C_6H_4Cl(4)$ | Cl | $OC_6H_{11}$-n | |
| 3.137 | $C_6H_4CN(4)$ | Cl | $OCH_3$ | |
| 3.138 | $C_6H_4F(3)$ | Cl | Cl | |
| 3.139 | $C_6H_4F(4)$ | Cl | $SCH_3$ | |
| 3.140 | $C_6H_4(OCH_3)(2)$ | Cl | Cl | |
| 3.141 | $C_6H_4(OCH_3)(3)$ | $SC_3H_7$-n | $C_4H_9$-t | |
| 3.142 | $C_6H_4(NO_2)(4)$ | Cl | $OC_6H_{11}$-n | |
| 3.143 | $C_6H_4[N(CH_3)_2](4)$ | Cl | Cl | |
| 3.144 | $C_6H_4(CF_3)(3)$ | Cl | Cl | |
| 3.145 | $C_6H_4(CF_3)(3)$ | Cl | $OCH_3$ | |
| 3.146 | $C_6H_4(CF_3)(4)$ | Cl | $SCH_3$ | |
| 3.147 | $C_6H_3Cl_2(2,4)$ | Cl | Cl | |
| 3.148 | $C_6H_3(NO_2)_2(3,5)$ | Cl | $OC_2H_5$ | |
| 3.149 | $C_6H_3Cl_2(2,4)$ | Cl | $OC_2H_5$ | |
| 3.150 | $C_6H_3(NO_2)(3,5)$ | Cl | Cl | |
| 3.151 | $C_6H_4(CH_3)(4)$ | Cl | Cl | |
| 3.152 | $C_6H_4(CH_3)(4)$ | Cl | $SCH_2CH{=}CH_2$ | |

Tabelle 3 (Fortsetzung)

| Verb. Nr. | $R_5$ | $R_6$ | $R_7$ | physikalische Konstante [°C] |
|---|---|---|---|---|
| 3.153 | $C_6H_4(C_4H_9\text{-t})(4)$ | Cl | Cl | |
| 3.154 | $SCH_3$ | H | $OC_3H_7\text{-n}$ | |
| 3.155 | $SCH_3$ | H | $O(CH_2)_2OCH_3$ | |
| 3.156 | $SCH_3$ | H | $O(CH_2)_3N(C_2H_5)_2$ | |
| 3.157 | $N(CH_3)_2$ | H | Cl | |
| 3.158 | $N(C_2H_5)_2$ | H | $OC_2H_5$ | |
| 3.159 | $N(CH_3)_2$ | H | $O(CH_2)_3Cl$ | |
| 3.160 | $C_6H_5$ | H | Cl | |
| 3.161 | $C_6H_5$ | H | $SC_2H_5$ | |
| 3.162 | $CH_3$ | Cl | $CH_3$ | |
| 3.163 | $CH_2C_6H_5$ | $OCH_3$ | $CH_3$ | |
| 3.164 | $C_6H_5$ | Cl | $CH_3$ | |
| 3.165 | $SCH_3$ | $OCH_2CF_3$ | $CF_3$ | |
| 3.166 | $CH_3$ | $CH_3$ | $CH_3$ | |
| 3.167 | $C_5H_{11}\text{-n}$ | $CH_3$ | $CH_3$ | |
| 3.168 | $C_6H_4(4)$ | $CF_3$ | $CF_3$ | |
| 3.169 | $C_6H_4CN(3)$ | $CF_3$ | $CF_3$ | |
| 3.170 | $N(CH_3)_2$ | $CH_3$ | $CH_3$ | |
| 3.171 | $SCH_3$ | H | $CH_3$ | |
| 3.172 | $C_2H_5$ | H | $CH_3$ | |
| 3.173 | $C_6H_4Cl(2)$ | H | $CH_3$ | |
| 3.174 | $CH_3$ | H | H | |

Tabelle 3 (Fortsetzung)

| Verb. Nr. | $R_5$ | $R_6$ | $R_7$ | physikalische Konstante [°C] |
|---|---|---|---|---|
| 3.175 | $SCH_3$ | H | H | |
| 3.176 | $C_6H_4(CF_3)(4)$ | H | H | |
| 3.177 | $C_6H_4(OCH_3)(3)$ | H | H | |
| 3.178 | H | Cl | $OC_2H_5$ | |
| 3.179 | H | Cl | $OCH(CH_3)C_2H_5$ | |
| 3.180 | H | Cl | O-Cyclohexyl | |
| 3.181 | H | Cl | $OCH_2CF_3$ | |
| 3.182 | H | Cl | $OCH_2CH=CH_2$ | Smp.87-88 |
| 3.183 | H | Cl | $OCH_2CH_2OCH_3$ | |
| 3.184 | H | Cl | $SCH_3$ | |
| 3.185 | H | Cl | $SCH_3$ | |
| 3.186 | H | Cl | $SCH(CH_3)_2$ | |
| 3.187 | H | Cl | SCN | |
| 3.188 | H | SCN | SCN | |
| 3.189 | H | Cl | $S(O)CH_3$ | |
| 3.190 | H | Cl | $SO_2CH_3$ | |
| 3.191 | H | SH | SH | |
| 3.192 | H | F | F | |
| 3.193 | H | $OCH(CF_3)_2$ | $OCH(CF_3)_2$ | |
| 3.194 | H | Cl | $O(CH_2)_3Cl$ | |
| 3.195 | H | $OCH_2CH_2NO_2$ | $O(CH_2)_3OC_2H_5$ | |
| 3.196 | H | Cl | $O(CH_2)_2N(C_4H_9\text{-}n)_2$ | |
| 3.197 | H | Cl | $OCH_2CH_2NO_2$ | |
| 3.198 | H | $OCH_3$ | $O(CH_2)_3N(CH_3)_2$ | |

Tabelle 3 (Fortsetzung)

| Verb. Nr. | $R_5$ | $R_6$ | $R_7$ | physikalische Konstante [°C] |
|---|---|---|---|---|
| 3.199 | H | $SC_2H_5$ | $OCH_3$ | |
| 3.200 | H | $SCH_2CH{=}CH_2$ | $N(C_4H_9\text{-}n)_2$ | |
| 3.201 | H | $N(CH_3)_2$ | $N(C_2H_5)_2$ | |
| 3.202 | H | F | $SCH_3$ | |
| 3.203 | H | $S\text{-}C_4H_9\text{-}t$ | $O(CH_2)_2OC_4H_9\text{-}n$ | |
| 3.204 | H | Cl | $OCH_2CH_2CN$ | |
| 3.205 | H | $OCH_3$ | $OCH_3$ | Smp. 172-174 |
| 3.206 | $CH_3$ | Cl | $OCH_3$ | Smp. 110-111 |
| 3.207 | $C_2H_5$ | Cl | $OCH_3$ | Smp. 98-99 |
| 3.208 | $C_2H_5$ | Cl | Cl | |
| 3.209 | $C_2H_5$ | Cl | $SCH_3$ | |
| 3.210 | Cl | H | $OCH_2CH_2SC_2H_5$ | |
| 3.211 | Cl | H | $OCH_2C{\equiv}CH$ | |
| 3.212 | Cl | H | $OCH_2CH{=}CH_2$ | |
| 3.213 | H | Cl | $OCH_2C{\equiv}CH$ | |
| 3.214 | $CH_3$ | Cl | $OCH_2CF_3$ | |
| 3.215 | $CH_3$ | Cl | $OCH_2CH{=}CH_2$ | |
| 3.216 | $CH_3$ | Cl | $OCH_2C{\equiv}CH$ | |
| 3.217 | $CH(CH_3)_2$ | Cl | Cl | |
| 3.218 | $CH(CH_3)_2$ | Cl | $OCH_3$ | |
| 3.219 | $CH(CH_3)_2$ | Cl | $SCH_3$ | |

Tabelle 3 (Fortsetzung)

| Verb. Nr. | $R_5$ | $R_6$ | $R_7$ | physikalische Konstante [°C] |
|---|---|---|---|---|
| 3.220 | $OCH_2CH{=}CH_2$ | Cl | H | |
| 3.221 | Cl | $CH_3$ | $SCH_3$ | |
| 3.222 | Cl | $CH_3$ | $OCH_2CH{=}CH_2$ | |
| 3.223 | $SCH_3$ | Cl | Cl | |
| 3.224 | $SCH_3$ | Cl | $OCH_3$ | |
| 3.225 | $SCH_3$ | Cl | $SCH_3$ | |
| 3.226 | $SCH_3$ | Cl | $OCH_2CF_3$ | |
| 3.227 | $SCH_3$ | Cl | $OCH_2CH{=}CH_2$ | |

Tabelle 4 : Verbindungen der Formel

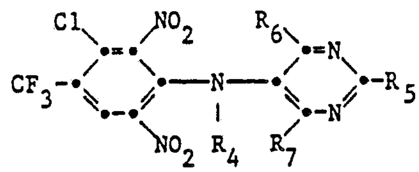

| Verb.Nr. | $R_5$ | $R_6$ | $R_7$ | $R_4$ | Phys. Konst.[°C] |
|---|---|---|---|---|---|
| 4.1 | H | Cl | $OCH_3$ | $C(O)CH_3$ | Smp. 181-182 |
| 4.2 | H | $SCH_3$ | $SCH_3$ | $C(O)CH_3$ | |
| 4.3 | H | $OCH_2CF_3$ | $OCH_2CF_3$ | $C(O)C_2H_5$ | |
| 4.4 | H | Cl | Cl | $C(O)CH_2Cl$ | |
| 4.5 | Cl | $CH_3$ | $CH_3$ | $C(O)CH_3$ | |
| 4.6 | SCN | $CH_3$ | $CH_3$ | $C(O)CH_2OCH_3$ | |
| 4.7 | $OCH_2CF_3$ | $CH_3$ | $CH_3$ | $C(O)CH_3$ | |
| 4.8 | $SCH_3$ | $CH_3$ | $CH_3$ | $C(O)C_2H_5$ | |
| 4.9 | $OC_2H_5$ | $CH_3$ | $CH_3$ | $C(O)CH_3$ | |
| 4.10 | Cl | $CF_3$ | $CF_3$ | $C(O)CH_3$ | |
| 4.11 | $OC_4H_9$-n | $CF_3$ | $CF_3$ | $C(O)CH_3$ | |
| 4.12 | $SCH_2CH=CH_2$ | $CF_3$ | $CF_3$ | $C(O)CH_2OC_2H_5$ | |
| 4.13 | F | $CF_3$ | $CF_3$ | $C(O)CH_2OCH_3$ | |
| 4.14 | $OCH(CH_3)_2$ | $CCl_3$ | $CCl_3$ | $C(O)CH_2OCH_3$ | |
| 4.15 | Cl | H | $CH_3$ | $C(O)CH_3$ | |
| 4.16 | Br | H | $CH_3$ | $C(O)CH_2Cl$ | |
| 4.17 | $OC_2H_5$ | H | $C_2H_5$ | $C(O)CH_2Cl$ | |
| 4.18 | SCN | H | $CH_3$ | $C(O)CH_3$ | |
| 4.19 | Cl | H | H | $C(O)CH_3$ | |

Tabelle 4 (Fortsetzung)

| Verb.Nr. | $R_5$ | $R_6$ | $R_7$ | $R_4$ | Physik. Konst.[°C] |
|---|---|---|---|---|---|
| 4.20 | $OCH_2CH_2F$ | H | H | $C(O)CH_3$ | |
| 4.21 | $O(CH_2)_3N(C_2H_5)_2$ | H | H | $C(O)CH_3$ | |
| 4.22 | $N(CH_3)_2$ | H | H | $C(O)CH_3$ | |
| 4.23 | $O(CH_2)_2SC_2H_5$ | H | H | $C(O)CH_3$ | |
| 4.24 | H | Cl | $OC_2H_5$ | $C(O)CH_3$ | Smp.108-109 |

Tabelle 5 : Verbindungen der Formel

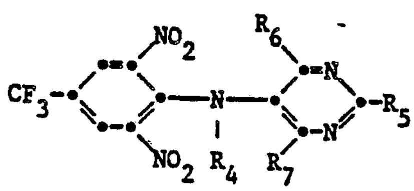

| Verb.Nr. | $R_5$ | $R_6$ | $R_7$ | $R_4$ |
|---|---|---|---|---|
| 5.1 | H | Cl | $OCH_3$ | $C(O)CH_3$ |
| 5.2 | H | $SCH_3$ | $SCH_3$ | $C(O)CH_3$ |
| 5.3 | H | $OCH_2CF_3$ | $OCH_2CF_3$ | $C(O)C_2H_5$ |
| 5.4 | H | Cl | Cl | $C(O)CH_2Cl$ |
| 5.5 | Cl | $CH_3$ | $CH_3$ | $C(O)CH_3$ |
| 5.6 | SCN | $CH_3$ | $CH_3$ | $C(O)CH_2OCH_3$ |
| 5.7 | $OCH_2CF_3$ | $CH_3$ | $CH_3$ | $C(O)CH_3$ |
| 5.8 | $SCH_3$ | $CH_3$ | $CH_3$ | $C(O)C_2H_5$ |

Tabelle 5 (Fortsetzung)

| Verb.Nr. | $R_5$ | $R_6$ | $R_7$ | $R_4$ |
|---|---|---|---|---|
| 5.9 | $OC_2H_5$ | $CH_3$ | $CH_3$ | $C(O)CH_3$ |
| 5.10 | Cl | $CF_3$ | $CF_3$ | $C(O)CH_3$ |
| 5.11 | $OC_4H_9$-n | $CF_3$ | $CF_3$ | $C(O)CH_3$ |
| 5.12 | $SCH_2CH{=}CH_2$ | $CF_3$ | $CF_3$ | $C(O)CH_2OC_2H_5$ |
| 5.13 | F | $CF_3$ | $CF_3$ | $C(O)CH_2OCH_3$ |
| 5.14 | $OCH(CH_3)_2$ | $CCl_3$ | $CCl_3$ | $C(O)CH_2OCH_3$ |
| 5.15 | Cl | H | $CH_3$ | $C(O)CH_3$ |
| 5.16 | Br | H | $CH_3$ | $C(O)CH_2Cl$ |
| 5.17 | $OC_2H_5$ | H | $C_2H_5$ | $C(O)CH_2Cl$ |
| 5.18 | SCN | H | $CH_3$ | $C(O)CH_3$ |
| 5.19 | Cl | H | H | $C(O)CH_3$ |
| 5.20 | $OCH_2CH_2F$ | H | H | $C(O)CH_3$ |
| 5.21 | $O(CH_2)_3N(C_2H_5)_2$ | H | H | $C(O)CH_3$ |
| 5.22 | $N(CH_3)_2$ | H | H | $C(O)CH_3$ |
| 5.23 | $O(CH_2)_2SC_2H_5$ | H | H | $C(O)CH_3$ |

Tabelle 6 : Verbindungen der Formel

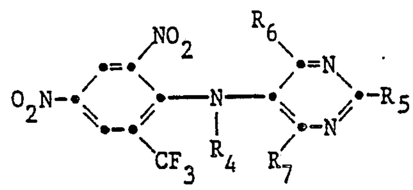

| Verb.Nr. | $R_5$ | $R_6$ | $R_7$ | $R_4$ | Physik. Konst.[°C] |
|---|---|---|---|---|---|
| 6.1 | H | Cl | $OCH_3$ | $C(O)CH_3$ | Smp. 167-169 |
| 6.2 | H | $SCH_3$ | $SCH_3$ | $C(O)CH_3$ | |
| 6.3 | H | $OCH_2CF_3$ | $OCH_2CF_3$ | $C(O)C_2H_5$ | |
| 6.4 | H | Cl | Cl | $C(O)CH_2Cl$ | |
| 6.5 | Cl | $CH_3$ | $CH_3$ | $C(O)CH_3$ | |
| 6.6 | SCN | $CH_3$ | $CH_3$ | $C(O)CH_2OCH_3$ | |
| 6.7 | $OCH_2CF_3$ | $CH_3$ | $CH_3$ | $C(O)CH_3$ | |
| 6.8 | $SCH_3$ | $CH_3$ | $CH_3$ | $C(O)C_2H_5$ | |
| 6.9 | $OC_2H_5$ | $CH_3$ | $CH_3$ | $C(O)CH_3$ | |
| 6.10 | Cl | $CF_3$ | $CF_3$ | $C(O)CH_3$ | |
| 6.11 | $OC_4H_9$-n | $CF_3$ | $CF_3$ | $C(O)CH_3$ | |
| 6.12 | $SCH_2CH=CH_2$ | $CF_3$ | $CF_3$ | $C(O)CH_2OC_2H_5$ | |
| 6.13 | F | $CF_3$ | $CF_3$ | $C(O)CH_2OCH_3$ | |
| 6.14 | $OCH(CH_3)_2$ | $CCl_3$ | $CCl_3$ | $C(O)CH_2OCH_3$ | |
| 6.15 | Cl | H | $CH_3$ | $C(O)CH_3$ | |
| 6.16 | Br | H | $CH_3$ | $C(O)CH_2Cl$ | |
| 6.17 | $OC_2H_5$ | H | $C_2H_5$ | $C(O)CH_2Cl$ | |
| 6.18 | SCN | H | $CH_3$ | $C(O)CH_3$ | |
| 6.19 | Cl | H | H | $C(O)CH_3$ | |

Tabelle 6 (Fortsetzung)

| Verb.Nr. | $R_5$ | $R_6$ | $R_7$ | $R_4$ |
|---|---|---|---|---|
| 6.20 | $OCH_2CH_2F$ | H | H | $C(O)CH_3$ |
| 6.21 | $O(CH_2)_3N(C_2H_5)_2$ | H | H | $C(O)CH_3$ |
| 6.22 | $N(CH_3)_2$ | H | H | $C(O)CH_3$ |
| 6.23 | $O(CH_2)_2SC_2H_5$ | H | H | $C(O)CH_3$ |

Tabelle 7 : Verbindungen der Formel

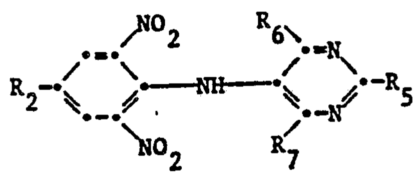

| Verb.Nr. | $R_5$ | $R_6$ | $R_7$ | $R_2$ | Smp.[°C] |
|---|---|---|---|---|---|
| 7.1 | H | Cl | Cl | $NO_2$ | 161-163 |
| 7.2 | Cl | Cl | H | $NO_2$ | 181-183 |
| 7.3 | H | Cl | $OCH_2CF_3$ | $NO_2$ | |
| 7.4 | H | $SCH_3$ | Cl | $NO_2$ | |
| 7.5 | Cl | H | H | $NO_2$ | |
| 7.6 | Cl | H | $OCH_3$ | $NO_2$ | |
| 7.7 | Cl | $SCH_3$ | H | $NO_2$ | |
| 7.8 | $CH_3$ | Cl | Cl | $NO_2$ | |
| 7.9 | $(CH_3)_2CH$ | $OCH_2C{\equiv}CH$ | Cl | $NO_2$ | |
| 7.10 | $CH_3$ | $OCH_2CH{=}CH_2$ | Cl | $NO_2$ | |
| 7.11 | $CH_3$ | Cl | $OCH(CH_3)_2$ | $NO_2$ | |
| 7.12 | Cl | Cl | $CH_3$ | $NO_2$ | |
| 7.13 | Cl | $OCH_3$ | $CH_3$ | $NO_2$ | |
| 7.14 | $SCH_3$ | Cl | Cl | $NO_2$ | |
| 7.15 | Cl | Cl | Cl | $NO_2$ | |
| 7.16 | F | F | H | $NO_2$ | |
| 7.17 | $C_2H_5$ | Cl | Cl | $NO_2$ | 130-131 |

Tabelle 8 : Verbindungen der Formel

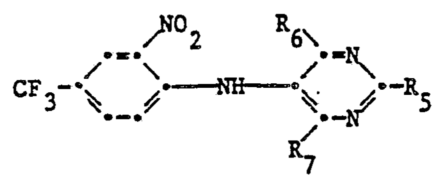

| Verb.Nr. | $R_5$ | $R_6$ | $R_7$ | Smp.[°C] |
|---|---|---|---|---|
| 8.1 | H | Cl | Cl | 119-121 |
| 8.2 | H | Cl | $OCH_3$ | 117-118 |
| 8.3. | Cl | Cl | H | 98 |
| 8.4 | Cl | Cl | $CH_3$ | |
| 8.5. | $CH_3$ | $OCH_3$ | Cl | |
| 8.6 | H | $OC_2H_5$ | Cl | |
| 8.7 | H | Cl | $SCH_3$ | |
| 8.8 | H | Cl | $OCH_2C{\equiv}CH$ | |
| 8.9 | H | Cl | $OCH_2CH{=}CH_2$ | Oel |
| 8.10 | $C_2H_5$ | $SCH_3$ | Cl | |
| 8.11 | $CH(CH_3)_2$ | $OC_4H_9$ | Cl | |
| 8.12 | $CH_3S$ | Cl | Cl | |
| 8.13 | Cl | Cl | Cl | |
| 8.14 | H | H | H | |
| 8.15 | H | Cl | $OCH_2CF_3$ | |
| 8.16 | Cl | Cl | $OCH(CH_3)_2$ | |
| 8.17 | $CH_3S$ | $OCH_3$ | Cl | |
| 8.18 | $CH_3$ | Cl | $SCH_3$ | |
| 8.19 | $CH_3$ | $OCH_2C{\equiv}CH$ | Cl | |
| 8.20 | H | Br | $OCH_3$ | |
| 8.21 | H | F | F | |
| 8.22 | $CH(CH_3)_2$ | $SCH_2CH{=}CH_2$ | Cl | |
| 8.23 | $CH(CH_3)_2$ | Cl | Cl | |
| 8.24 | $C_2H_5$ | Cl | $OCH_3$ | |

Tabelle 8 (Fortsetzung)

| Verb.Nr. | $R_5$ | $R_6$ | $R_7$ | Smp.[°C] |
|---|---|---|---|---|
| 8.25 | $C_2H_5$ | Cl | $OCH_2CH=CH_2$ | |
| 8.26 | $CH_3$ | $OCH(CH_3)_2$ | $OCH_3$ | |
| 8.27 | $CH_3$ | $SCH_3$ | $OC_2H_5$ | |
| 8.28 | H | Cl | $OC(CH_3)_3$ | 111 |
| 8.29 | Cl | H | $OCH_3$ | |
| 8.30 | Cl | H | $OCH_2$ | |
| 8.31 | $CH(CH_3)_2$ | Cl | $OCH_3$ | |
| 8.32 | $CH(CH_3)_2$ | Cl | $SCH_3$ | |
| 8.33 | $SCH_3$ | Cl | $OCH_2CF_3$ | |
| 8.34 | $SCH_3$ | Cl | $OCH_2CH=CH_2$ | |
| 8. 5 | $SCH_3$ | Cl | $OCH_2C\equiv CH$ | |

Tabelle 9 : Verbindungen der Formel

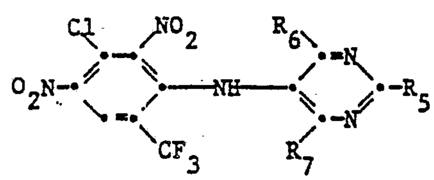

| Verb.Nr. | $R_5$ | $R_6$ | $R_7$ | Smp.[°C] |
|---|---|---|---|---|
| 9.1 | H | Cl | $SCH_3$ | 172-176 |
| 9.2 | H | $OCH_3$ | H | 108-110 |
| 9.3 | H | $OCH_3$ | $OCH_3$ | 196-199 |
| 9.4 | $CH_3$ | $OC_2H_5$ | H | 124-126 |
| 9.5 | Cl | H | $OCH_3$ | 143-146 |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykol-ether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| F3. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit den Träger vermischt auf einer geeigneten Mühle vermahlen wird.

F8. Extruder Granulat

| | |
|---|---|
| Wirkstoff aus den Tabellen | 10 % |
| N-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

F9. Umhüllungs-Granulat

| | |
|---|---|
| Wirkstoff aus den Tabellen | 3 % |
| Polyethylenglykol (M G 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37 %ige wässrige Formaldehyd-Lösung | 0,2 % |
| Silikonöl in Form einer 75 % igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel B1 : Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,02 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus der Tabelle zeigten gegen Puccinia-Pilze eine sehr gute Wirkung. Unbehandelte aber infizierte Kontrollpflanzen zeigten einen Puccinia-Befall von 100 %. Unter anderen hemmten die Verbindungen Nr. 1.1 bis 1.4, 1.19, 1.49, 1.51, 1.57, 1.78, 1.96, 1.111, 1.125, 1.151, 1.178, 1.184, 1.205, 1.207, 1.210, 1.211, 1.213, 1.214, 1.216, 1.218, 1.220, 2.1, 2.4, 2.49, 2.51, 2.96, 2.207, 2.208, 2.210, 2.211, 2.212, 3.1, 3.4, 3.49, 3.51, 3.206, 4.1, 4.24, 6.1, 7.1, 7.2, 8.1, 8.2, 8.3, 8.28 und 9.1 den Puccinia-Befall auf 0 bis 5 %.

### Beispiel B2 : Wirkung gegen Cersopora arachidicola auf Erdnusspflanzen

Residual-protektive Wirkung

10-15 cm hohe Erdnusspflanzen wurden mit einer aus Spritzpulver der Wirksubstanz hergestellten Spritzbrühe (0,006 Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen behandelt wurden, einen stark reduzierten Cercospora-Befall. So verhinderten die Verbindungen Nr. 1.1 bis 1.4, 1.19, 1.49, 1.78, 1.96, 1.111, 1.125, 1.126, 1.151, 1.178, 1.184, 1.205, 1.206, 1.207, 1.213, 1.216, 1.218, 1.220, 2.1, 2.4, 2.49, 2.51, 2.96, 2.211, 2.212, 2.1, 2.4, 3.49, 3.51, 3.207, 4.1, 4.24, 6.1, 7.1, 7.2, 8.1, 8.3, 8.28 und 9.1 in den obigen Versuchen das Auftreten von Flecken fast vollständig (0-10 %).

### Beispiel B3 : Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,002 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen zeigten einen Erysiphe-Befall von 100 %. Einzelne Verbindungen aus den Tabellen hemmten den Pilzbefall bei Gerste auf weiniger als 30 %.

Beispiel B4 : Residual-protektive Wirkung gegen Venturia inaequalis auf Apfeltrieben.

Apfelstecklinge mit 10-20 cm langen Frischtrieben wurden mit einer aus Spritzpulver des Wirkstoffes hergestellten Spritzbrühe (0,006 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt. Verbindungen aus den Tabellen, so z. B. Nr. 1.1, 1.51, 1.126, 1.127, 1.178, 1.184, 1.205, 1.206, 1.210, 1.211, 1.215, 1.217, 1.218, 2.4, 2.51, 2.96, 2.207, 2.208, 2.210, 2.211, 3.1, 3.4, 3.205, 3.206, 4.1, 4.24, 6.1, 7.17, 8.1 und 8.2 hemmten den Krankheitsbefall auf 25 % und weniger. Unbehandelte aber infizierte Kontrollbetriebe wurden dagegen 100 %ig befallen.

Beispiel B5 : Wirkung gegen Botrytis cinerea auf Bohnen

Residual protektive Wirkung

Ca. 10 cm hohe Bohnen-Pflanzen wurden mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21 °C erfolgte die Beurteilung des Pilzbefalls. Die Verbindungen aus der Tabelle hemmten in vielen Fällen die Pilzinfektion sehr stark. Bei einer Konzentration von 0,02 % erwiesen sich z. B. die Verbindungen 1.1 bis 1.4, 1.49, 1.51, 1.57, 1.196, 1.184, 1.210, 1.211 und 2.4 als voll wirksam. Der Krankheitsbefall lag bei 0 bis 8 %. Der Botrytis-Befall unbehandelter aber infizierter Bohnenpflanzen betrug 100 %.

Beispiel B6 : Wirkung gegen Phytophthora infestans auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,06 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

b) Systemische Wirkung

Zu Tomatenpflanzen wurde nach 3-wöchiger Anzucht eine aus Spritzpulver des Wirkstoffes hergestellte Spritzbrühe gegossen (0,06 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

Unter anderen zeigten in obigen Versuchen die Verbindungen Nr. 1.1 bis 1.4, 1.19, 1.49, 1.51, 1.57, 1.78, 1.96, 1.111, 1.125, 1.151, 1.178, 1.184, 1.205, 1.207, 1.210, 1.211, 1.213, 1.214, 1.216, 1.218, 1.220, 2.1, 2.4, 2.49, 2.51, 2.96, 2.207, 2.208, 2.210, 2.211, 2.212, 3.1, 3.4, 3.49, 3.51, 3.206, 4.1, 4.24, 6.1, 7.1, 7.2, 8.1, 8.2, 8.3, 8.28 und 9.1 eine sehr gute systemische Wirkung. Gegenüber unbehandelten Kontrollpflanzen (100 % Befall) bewirkten diese Verbindungen eine fast vollständige Unterdrückung des Pilzbefalls (0 bis 5 %).

Beispiel B7 : Wirkung gegen Plasmapora viticola auf Reben

a) Residual-protektive Wirkung

Im 4-5 Blattstadium wurden Rebensämlinge mit einer aus Spritzpulver des Wirkstoffes hergestellten Spritzbrühe (0,06 % Aktivsubstanz) besprüht. Nach 24 Stunden wurden die behandelten Pflanzen mit einer Sporangiensuspension des Pilzes infiziert. Nach einer Inkubation während 6 Tagen bei 95-100 %

relativer Luftfeuchtigkeit und 20 °C wurde der Pilzbefall beurteilt.

b) Residual-kurative Wirkung

Im 4-5 Blattstadium wurden Rebensämlinge mit einer Sporangiensuspension des Pilzes infiziert. Nach einer Inkubation während 24 Stunden in einer Feuchtkammer bei 95-100 % relativer Luftfeuchtigkeit und 20 °C wurden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 6 Tage nach der Infektion.

Verbindungen aus den Tabellen zeigten gegen Plasmopara viticola auf Regen eine sehr gute fungizide Wirkung, so z. B. unter anderen die Verbindungen Nr. 1.1, 1.2, 1.3 und 1.4 eine vollständige Unterdrückung des Pilzbefalls (0 bis 5 %).

Beispiel B8 : Wirkung gegen Piricularia oryzae auf Reispflanzen

Residual-protektive Wirkung

Reispflanzen wurden nach zweiwöchiger Anzucht mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach 5 Tagen Inkubation bei 95-100 % relativer Luftfeuchtigkeit und 24 °C wurde der Pilzbefall beurteilt.

Reispflanzen, die mit einer Spritzbrühe behandelt worden sind, die als Aktivsubstanz eine Verbindung 1.1, 1.2, 1.3, 1.4, 1.51, 1.96, 1.205, 2.4, 3.51 und 8.1 enthielt, zeigten im Vergleich zu unbehandelten Kontrollpflanzen (100 % Befall) weniger als 10 % Pilzbefall.

**Patentansprüche** (für die Vertragsstaaten : GB, BE, DE, FR, IT, LU, NL, SE, CH, LI)

1. Verbindungen der allgemeinen Formel I

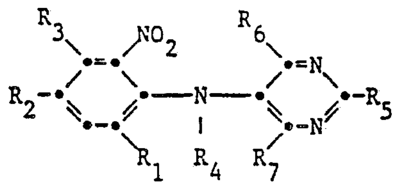

(I)

worin

$R_1$ und $R_2$ unabhängig voneinander für $NO_2$ oder $CF_3$ stehen oder einer von beiden für Wasserstoff steht ;

$R_3$ Wasserstoff oder Halogen bedeutet ;

$R_4$ für Wasserstoff oder die Gruppe —C(O)R′ steht, wobei R′ unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$, $R_6$ und $R_7$ unabhängig voneinander für Wasserstoff, Halogen, Nitro, Cyano, Rhodano, Mercapto, $C_1$-$C_6$-Alkyl, $C_1$-$C_6$-Haloalkyl, $C_1$-$C_6$-Alkoxy, durch Halogen, Nitro, Cyano, $C_1$-$C_3$-Alkoxy, $C_1$-$C_3$-Alkylthio und/oder $N(C_1$-$C_4$-alkyl$)_2$ substituiertes $C_1$-$C_6$-Alkoxy, $C_3$-$C_6$-Cycloalkoxy, $C_3$-$C_6$-Alkenyloxy, $C_3$-$C_6$-Alkinyloxy, $C_1$-$C_6$-Alkylthio, $C_3$-$C_6$-Alkenylthio, $C_1$-$C_6$-Alkylsulfonyl, $C_1$-$C_6$-Alkylsulfoxyl, $C_3$-$C_6$-Alkenyl $C_3$-$C_6$-Haloalkenyl, $C_3$-$C_6$-Alkinyl, $C_3$-$C_6$-Haloalkinyl oder für die Gruppe $N(C_1$-$C_4$-alkyl$)_2$ stehen und $R_5$ zusätzlich eine unsubstituierte oder ein- bis dreifach durch Halogen, Nitro, Cyano, $C_1$-$C_4$-Alkyl, $C_1$-$C_3$-Haloalkyl, $N(C_1$-$C_3$-alkyl$)_2$ und/oder $C_1$-$C_3$-Alkoxy substituierte Phenyl- oder Benzylgruppe sein kann.

2. Verbindungen der Formel I nach Anspruch 1, dadurch gekennzeichnet, dass

$R_1$ für $NO_2$ oder $CF_3$ steht ;

$R_2$ für $NO_2$ oder $CF_3$ steht ;

$R_3$ Wasserstoff oder Halogen bedeutet ;

$R_4$ für Wasserstoff oder die Gruppe —C(O)R′ steht, wobei R′ unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$, $R_6$ und $R_7$ unabhängig voneinander für Wasserstoff, Halogen, Nitro, Cyano, Rhodano, Mercapto, $C_1$-$C_6$-Alkyl, $C_1$-$C_6$-Haloalkyl, $C_1$-$C_6$-Alkoxy, durch Halogen, Nitro, Cyano, $C_1$-$C_3$-Alkoxy, $C_1$-$C_3$-Alkylthio und/oder $N(C_1$-$C_4$-alkyl$)_2$ substituiertes $C_1$-$C_6$-Alkoxy, $C_3$-$C_6$-Cycloalkoxy, $C_3$-$C_6$-Alkenyloxy, $C_3$-$C_6$-Alkinyloxy, $C_1$-$C_6$-Alkylthio, $C_3$-$C_6$-Alkenylthio, $C_1$-$C_6$-Alkylsulfonyl, $C_1$-$C_6$-Alkylsulfoxyl oder für die Gruppe $N(C_1$-$C_4$-alkyl$)_2$ stehen und $R_5$ zusätzlich eine unsubstituierte oder ein- bis dreifach durch Halogen, Nitro, Cyano, $C_1$-$C_4$-Alkyl, $C_1$-$C_3$-Haloalkyl, $N(C_1$-$C_3$-alkyl$)_2$ und/oder $C_1$-$C_3$-Alkoxy substituierte Phenyl- oder Benzylgruppe sein kann.

3. Verbindungen der Formel I nach Anspruch 2, dadurch gekennzeichnet, dass $R_1$ für Nitro, $R_2$ für Trifluormethyl, $R_3$ für Chlor stehen, $R_4$ Wasserstoff oder die Gruppe —C(O)R′ bedeutet, wobei R′

unsubstituiertes oder durch Halogen, $C_1$-$C_3$-Alkoxy oder $C_1$-$C_3$-Alkylthio substituiertes $C_1$-$C_4$-Alkyl bedeutet ; $R_5$ Wasserstoff, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$ Alkylthio, durch N($C_1$-$C_2$-alkyl)$_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, N($C_1$-$C_3$-alkyl)$_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind ; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder N($C_1$-$C_4$-alkyl)$_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenyloxy, $C_3$-$C_4$-Alkenylthio, $C_3$-$C_4$-Alkinyloxy, $C_3$-$C_4$-Alkinylthio oder N($C_1$-$C_3$-alkyl)$_2$ stehen.

4. Verbindungen der Formel I nach Anspruch 3, dadurch gekennzeichnet, dass $R_4$ für Wasserstoff steht und die übrigen Substituenten wie in Anspruch 3 definiert sind.

5. Verbindungen der Formel I nach Anspruch 2, dadurch gekennzeichnet, dass $R_1$ für Nitro, $R_2$ für Trifluormethyl, $R_3$ für Wasserstoff stehen, $R_4$ Wasserstoff oder die Gruppe —C(O)R′ bedeutet, wobei R′ unsubstituiertes oder durch Halogen, $C_1$-$C_3$-Alkoxy oder $C_1$-$C_3$-Alkylthio substituiertes $C_1$-$C_4$-Alkyl bedeutet ; $R_5$ Wasserstoff, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$-Alkylthio, durch N($C_1$-$C_2$-alkyl)$_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, N($C_1$-$C_3$-alkyl)$_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind ; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder N($C_1$-$C_4$-alkyl)$_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenyloxy, $C_3$-$C_4$-Alkenylthio, $C_3$-$C_4$-Alkinyloxy, $C_3$-$C_4$-Alkinylthio oder N($C_1$-$C_3$-alkyl)$_2$ stehen.

6. Verbindungen der Formel I nach Anspruch 5, dadurch gekennzeichnet, dass $R_4$ für Wasserstoff steht und die übrigen Substituenten wie in Anspruch 5 definiert sind.

7. Verbindungen der Formel I nach Anspruch 2, dadurch gekennzeichnet, dass $R_1$ für Trifluormethyl, $R_2$ für Nitro, $R_3$ für Wasserstoff stehen, $R_4$ Wasserstoff oder die Gruppe —C(O)R′ bedeutet, wobei R′ unsubstituiertes oder durch Halogen, $C_1$-$C_3$-Alkoxy oder $C_1$-$C_3$-Alkylthio substituiertes $C_1$-$C_4$-Alkyl bedeutet ; $R_5$ Wasserstoff, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$-Alkylthio, durch N($C_1$-$C_2$-alkyl)$_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, N($C_1$-$C_3$-alkyl)$_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind ; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder N($C_1$-$C_4$-alkyl)$_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenyloxy, $C_3$-$C_4$-Alkenylthio, $C_3$-$C_4$-Alkinyloxy, $C_3$-$C_4$-Alkinylthio oder N($C_1$-$C_3$-alkyl)$_2$ stehen.

8. Verbindungen der Formel I nach Anspruch 7, dadurch gekennzeichnet, dass $R_4$ für Wasserstoff steht und die übrigen Substituenten wie in Anspruch 7 definiert sind.

9. Eine Verbindung der Formel I nach Anspruch 1, ausgewählt aus der Reihe :
N-(3′-Chlor-2′,6′-dinitro-4′-trifluormethylphenyl)-5-amino-4-chlor-6-methoxypyrimidin (Nr. 1.1),
N-(3′-Chlor-2′,6′-dinitro-4′-trifluormethylphenyl)-5-amino-4,6-dimethylthiopyrimidin (Nr. 1.2),
N-(3′-Chlor-2′,6′-dinitro-4′-trifluormethylphenyl)-5-amino-4,6-ditrifluorethoxypyrimidin (Nr. 1.3),
N-(3′-Chlor-2′,6′-dinitro-4′-trifluormethylphenyl)-5-amino-4,6-dichlorpyrimidin (Nr. 1.4),
N-(3′-Chlor-2′,6′-dinitro-4′-trifluormethylphenyl)-5-amino-4-chlor-6-methylmercaptopyrimidin (Nr. 1.184) ;
N-(3′-Chlor-2′,6′-dinitro-4′-trifluormethylphenyl)-5-amino-4-chlor-2-methyl-6-methoxypyrimidin (Nr. 1.205) ;
N-(3-Chlor-2′,6′-dinitro-4′-trifluormethylphenyl)-5-amino-2-chlor-4-methoxypyrimidin (Nr. 1.51) ;
N-(3′-Chlor-2′,6′-dinitro-4′-trifluormethylphenyl)-5-amino-2,4-dichlorpyrimidin (Nr. 1.49) ;
N-(3′-Chlor-2′,6′-dinitro-4′-trifluormethylphenyl)-5-amino-2-chlor-4-allyloxypyrimidin (Nr. 1.210) ;
N-(3′-Chlor-2′,6′-dinitro-4′-trifluormethylphenyl)-5-amino-2-chlor-4-allylmercaptopyrimidin (Nr. 1.57) ;
N-(3′-Chlor-2′,6′-dinitro-4′-trifluormethylphenyl)-5-amino-2-chlor-4-propargyloxypyrimidin (Nr. 1.211) ;
N-(3′-Chlor-2′,6′-dinitro-4′-trifluormethylphenyl)-5-amino-4,6-dichlor-2-methylpyrimidin (Nr. 1.96) ;
N-(3′-Chlor-2′,6′-dinitro-4′-trifluormethylphenyl)-5-amino-4,6-dichlor-2-phenylpyrimidin (Nr. 1.125) ;
N-(2′,6′-Dinitro-4′-trifluormethylphenyl)-5-amino-4,6-dichlorpyrimidin (Nr. 2.4) ;
N-(2′,4-Dinitro-6′-trifluormethylphenyl)-5-amino-4,6-dichlorpyrimidin (Nr. 3.4) ;
N-(2′,4′,6′-Trinitrophenyl)-5-amino-4,6-dichlorpyrimidin (Nr. 7.1) ;
N-(2′-Nitro-4′-trifluormethylphenyl)-5-amino-4,6-dichlorpyrimidin (Nr. 8.1).

10. Verfahren zur Herstellung von Verbindungen der Formel I, dadurch gekennzeichnet, dass man eine Verbindung der Formel II

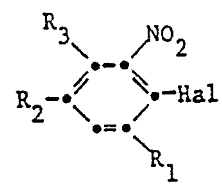

(II)

in Gegenwart einer Base mit einem Pyrimidin-Derivat der Formel III

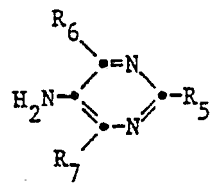

(III)

zu einer Verbindung der Formel I'

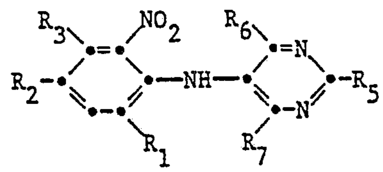

(I')

umsetzt, und letztere zur Herstellung N-acylierter Derivate mit einem reaktionsfähigen Derivat der Carbonsäure der Formel IV

$$R_4COOH \qquad (IV)$$

N-acyliert, wobei die Substituenten $R_1$ bis $R_7$ die unter Formel I angegebenen Bedeutungen haben und Hal für Halogen steht.

11. Mittel zur Bekämpfung oder Verhütung eines Befalls durch Schädlinge, dadurch gekennzeichnet, dass es als mindestens eine aktive Komponente eine Verbindung der Formel I enthält sowie übliche Zuschlagsstoffe und Trägermaterialien.

12. Verfahren zur Bekämpfung oder Verhütung eines Befalls von Kulturpflanzen durch phytopathogene Schädlinge, dadurch gekennzeichnet, dass man eine Verbindung der Formel I auf die Pflanze oder deren Standort appliziert.

**Patentansprüche** (für den Vertragsstaat AT)

1. Verfahren zur Herstellung von Verbindungen der Formel I

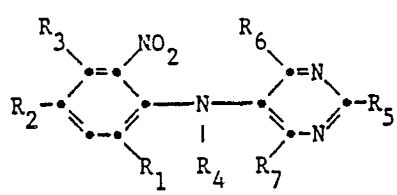

(I)

worin

$R_1$ und $R_2$ unabhängig voneinander für $NO_2$ oder $CF_3$ stehen oder einer von beiden für Wasserstoff steht ;

$R_3$ Wasserstoff oder Halogen bedeutet ;

$R_4$ für Wasserstoff oder die Gruppe —C(O)R' steht, wobei R' unsubstituiertes oder durch Halogen, $C_1$-$C_3$-Alkoxy oder $C_1$-$C_3$-Alkylthio substituiertes $C_1$-$C_4$-Alkyl bedeutet ;

$R_5$, $R_6$ und $R_7$ unabhängig voneinander für Wasserstoff, Halogen, Nitro, Cyano, Rhodano, Mercapto, $C_1$-$C_6$-Alkyl, $C_1$-$C_6$-Haloalkyl, $C_1$-$C_6$-Alkoxy, durch Halogen, Nitro, Cyano, $C_1$-$C_3$-Alkoxy, $C_1$-$C_3$-Alkylthio und/oder $N(C_1$-$C_4$-alkyl$)_2$ substituiertes $C_1$-$C_6$-Alkoxy, $C_3$-$C_6$-Cycloalkoxy, $C_3$-$C_6$-Alkenyloxy, $C_3$-$C_6$-Alkinyloxy, $C_1$-$C_6$-Alkylthio, $C_3$-$C_6$-Alkenylthio, $C_1$-$C_6$-Alkylsulfonyl, $C_1$-$C_6$-Alkylsulfoxyl, $C_3$-$C_6$-Alkenyl, $C_3$-$C_6$-Haloalkenyl, $C_3$-$C_6$-Alkinyl, $C_3$-$C_6$-Haloalkinyl oder für die Gruppe $N(C_1$-$C_4$-alkyl$)_2$ stehen und $R_5$ zusätzlich eine unsubstituierte oder ein- bis dreifach durch Halogen, Nitro, Cyano, $C_1$-$C_4$-Alkyl, $C_1$-$C_3$-

Haloalkyl, $N(C_1-C_3\ alkyl)_2$, und/oder $C_1$-$C_3$-Alkoxy substituierte Phenyl- oder Benzylgruppe sein kann, dadurch gekennzeichnet, dass man eine Verbindung der Formel II

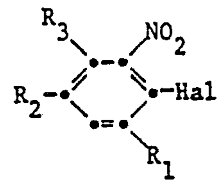

(II)

in Gegenwart einer Base mit einem Pyrimidin-Derivat der Formel III

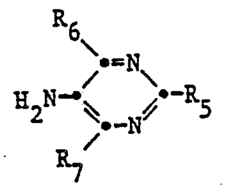

(III)

zu einer Verbindung der Formel I'

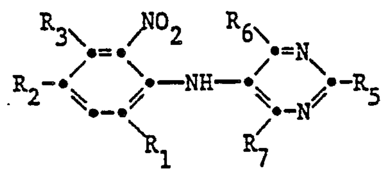

(I')

umsetzt, und letztere zur Herstellung N-acylierter Derivate mit einem reaktionsfähigen Derivat der Carbonsäure der Formel IV

$$R_4COOH \qquad (IV)$$

N-acyliert, wobei die Substituenten $R_1$ bis $R_7$ die unter Formel I angegebenen Bedeutungen haben und Hal für Halogen steht.

2. Verfahren nach Anspruch 1, zur Herstellung von Verbindungen der Formel I, worin
$R_1$ für $NO_2$ oder $CF_3$ steht ;
$R_2$ für $NO_2$ oder $CF_3$ steht ;
$R_3$ Wasserstoff oder Halogen bedeutet ;
$R_4$ für Wasserstoff oder die Gruppe —C(O)R' steht, wobei R' unsubstituiertes oder durch Halogen, $C_1$-$C_3$-Alkoxy oder $C_1$-$C_3$-Alkylthio substituiertes $C_1$-$C_4$-Alkyl bedeutet ;
$R_5$, $R_6$ und $R_7$ unabhängig voneinander für Wasserstoff, Halogen, Nitro, Cyano, Rhodano, Mercapto, $C_1$-$C_6$-Alkyl, $C_1$-$C_6$-Haloalkyl, $C_1$-$C_6$-Alkoxy, durch Halogen, Nitro, Cyano, $C_1$-$C_3$-Alkoxy, $C_1$-$C_3$-Alkylthio und/oder $N(C_1-C_4\text{-alkyl})_2$ substituiertes $C_1$-$C_6$-Alkoxy, $C_3$-$C_6$-Cycloalkoxy, $C_3$-$C_6$-Alkenyloxy, $C_3$-$C_6$-Alkinyloxy, $C_1$-$C_6$-Alkylthio, $C_3$-$C_6$-Alkenylthio, $C_1$-$C_6$-Alkylsulfonyl, $C_1$-$C_6$-Alkylsulfoxyl oder für die Gruppe $N(C_1-C_4\text{-alkyl})_2$ stehen und $R_5$ zusätzlich eine unsubstituierte oder ein- bis dreifach durch Halogen, Nitro, Cyano, $C_1$-$C_4$-Alkyl, $C_1$-$C_3$-Haloalkyl, $N(C_1-C_3\text{-alkyl})_2$ und/oder $C_1$-$C_3$-Alkoxy substituierte Phenyl- oder Benzylgruppe sein kann.

3. Verfahren nach Anspruch 2, zur Herstellung von Verbindungen der Formel I, worin $R_1$ für Nitro, $R_2$ für Trifluormethyl, $R_3$ für Chlor stehen, $R_4$ Wasserstoff oder die Gruppe —C(O)R' bedeutet, wobei R' unsubstituiertes oder durch Halogen, $C_1$-$C_3$-Alkoxy oder $C_k$-$C_3$-Alkylthio substituiertes $C_1$-$C_4$-Alkyl bedeutet ; Alkyl bedeutet ; $R_5$ Wasserstoff, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$ Alkylthio, durch $N(C_1-C_2\text{-alkyl})_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, $N(C_1-C_3\text{-alkyl})_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind ; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder $N(C_1-C_4\text{-alkyl})_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenyloxy, $C_3$-$C_4$-Alkenylthio, $C_3$-$C_4$-Alkinyloxy, $C_3$-$C_4$-Alkinylthio oder $N(C_1-C_3\text{-alkyl})_2$ stehen.

4. Verfahren nach Anspruch 3, zur Herstellung von Verbindungen der Formel I, worin $R_4$ für Wasserstoff steht und die übrigen Substituenten wie in Anspruch 3 definiert sind.

5. Verfahren nach Anspruch 2, zur Herstellung von Verbindungen der Formel I nach Anspruch 1, worin $R_1$ für Nitro, $R_2$ für Trifluormethyl, $R_3$ für Wasserstoff stehen, $R_4$ Wasserstoff oder die Gruppe —C(O)R' bedeutet, wobei R' unsubstituiertes oder durch Halogen, $C_1$-$C_3$-Alkoxy oder $C_1$-$C_3$-Alkylthio substituiertes $C_1$-$C_4$-Alkyl bedeutet; $R_5$H, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$-Alkylthio, durch N($C_1$-$C_2$-alkyl)$_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, N($C_1$-$C_3$-alkyl)$_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder N($C_1$-$C_4$-alkyl)$_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenyloxy, $C_3$-$C_4$-Alkenylthio, $C_3$-$C_4$-Alkinyloxy, $C_3$-$C_4$-Alkinylthio oder N($C_1$-$C_3$-alkyl)$_2$ stehen.

6. Verfahren nach Anspruch 5, zur Herstellung von Verbindungen der Formel I, worin $R_4$ für Wasserstoff steht und die übrigen Substituenten wie in Anspruch 5 definiert sind.

7. Verfahren nach Anspruch 2, zur Herstellung von Verbindungen der Formel I, worin $R_1$ für Trifluormethyl, $R_2$ für Nitro, $R_3$ für Wasserstoff stehen, $R_4$ Wasserstoff oder die Gruppe —C(O)R' bedeutet, wobei R' unsubstituiertes oder durch Halogen, $C_1$-$C_3$-Alkoxy oder $C_1$-$C_3$-Alkylthio substituiertes $C_1$-$C_4$-Alkyl bedeutet;

$R_5$ Wasserstoff, Halogen, Cyano, Rhodano, $C_1$-$C_6$-Alkyl, $C_1$-$C_3$-Haloalkyl, $C_1$-$C_6$-Alkoxy, $C_1$-$C_3$-Haloalkoxy, $C_1$-$C_3$-Alkylthio-($C_1$-$C_3$-alkoxy), $C_1$-$C_3$-Alkylthio, durch N($C_1$-$C_2$-alkyl)$_2$ substituiertes $C_1$-$C_3$-Alkoxy, $C_3$-$C_4$-Alkenyloxy, $C_3$-$C_4$-Alkenylthio, $C_1$-$C_3$-Alkylsulfonyl, $C_1$-$C_3$-Alkylsulfoxyl, N($C_1$-$C_3$-alkyl)$_2$ oder eine Phenyl- oder Benzylgruppe bedeutet, die beide unsubstituiert oder ein- bis dreifach durch Fluor, Chlor, Brom, Nitro, Cyano, Methoxy, $C_1$-$C_4$-Alkyl, Dimethylamino oder $CF_3$ substituiert sind; und $R_6$ und $R_7$ unabhängig voneinander für Wasserstoff, Halogen, Cyano, Rhodano, $C_1$-$C_6$-Alkyl, $C_1$-$C_3$-Haloalkyl, $C_1$-$C_6$-Alkoxy, durch Halogen, Nitro, $C_1$-$C_3$-Alkoxy, $C_1$-$C_3$-Alkylthio oder N($C_1$-$C_4$-alkyl)$_2$ substituiertes $C_1$-$C_3$-Alkoxy, $C_1$-$C_4$-Alkylthio, $C_1$-$C_3$-Alkylsulfonyl, $C_1$-$C_3$-Alkylsulfoxyl, $C_3$-$C_4$-Alkenyloxy, $C_3$-$C_4$-Alkenylthio, $C_3$-$C_4$-Alkinyloxy, $C_3$-$C_4$-Alkinylthio oder N($C_1$-$C_3$-alkyl)$_2$ stehen.

8. Verfahren nach Anspruch 7, zur Herstellung von Verbindungen der Formel I, worin $R_4$ für Wasserstoff steht und die übrigen Substituenten wie in Anspruch 7 definiert sind.

9. Verfahren nach Anspruch 1, zur Herstellung einer Verbindung der Formel I, ausgewählt aus der Reihe:

N-(3'-Chlor-2',6'-dinitro-4'-trifluormethylphenyl)-5-amino-4-chlor-6-methoxypyrimidin (Nr. 1.1),
N-(3'-Chlor-2',6'-dinitro-4'-trifluormethylphenyl)-5-amino-4,6-dimethylthiopyrimidin (Nr. 1.2),
N-(3'-Chlor-2',6'-dinitro-4'-trifluormethylphenyl)-5-amino-4,6-ditrifluorethoxypyrimidin (Nr. 1.3),
N-(3'-Chlor-2',6'-dinitro-4'-trifluormethylphenyl)-5-amino-4,6-dichlorpyrimidin (Nr. 1.4),
N-(3'-Chlor-2',6'-dinitro-4'-trifluormethylphenyl)-5-amino-4-chlor-6-methylmercaptopyrimidin (Nr. 1.184);
N(3'-Chlor-2',6'-dinitro-4'-trifluormethylphenyl)-5-amino-4-chlor-2-methyl-6-methoxypyrimidin (Nr. 1.205);
N-(3-Chlor-2',6'-dinitro-4'-trifluormethylphenyl)-5-amino-2-chlor-4-methoxypyrimidin (Nr. 1.51);
N-(3'-Chlor-2',6'-dinitro-4'-trifluormethylphenyl)-5-amino-2,4-dichlorpyrimidin (Nr. 1.49);
N-(3'-Chlor-2',6'-dinitro-4'-trifluormethylphenyl)-5-amino-2-chlor-4-allyloxypyrimidin (Nr. 1.210);
N-(3'-Chlor-2',6'-dinitro-4'-trifluormethylphenyl)-5-amino-2-chlor-4-allylmercaptopyrimidin (Nr. 1.57);
N-(3'-Chlor-2',6'-dinitro-4'-trifluormethylphenyl)-5-amino-2-chlor-4-propargyloxypyrimidin (Nr. 1.211);
N-(3'-Chlor-2',6'-dinitro-4'-trifluormethylphenyl)-5-amino-4,6-dichlor-2-methylpyrimidin (Nr. 1.96);
N-(3'-Chlor-2',6'-dinitro-4'-trifluormethylphenyl)-5-amino-4,6-dichlor-2-phenylpyrimidin (Nr. 1.125);
N-(2',6'-Dinitro-4'-trifluormethylphenyl)-5-amino-4,6-dichlorpyrimidin (Nr. 2.4);
N-(2',4-Dinitro-6'-trifluormethylphenyl)-5-amino-4,6-dichlorpyrimidin (Nr. 3.4);
N-(2',4',6'-Trinitrophenyl)-5-amino-4,6-dichlorpyrimidin (Nr. 7.1);
N-(2'-Nitro-4'-trifluormethylphenyl)-5-amino-4,6-dichlorpyrimidin (Nr. 8.1).

10. Mittel zur Bekämpfung oder Verhütung eines Befalls durch Schädlinge, dadurch gekennzeichnet, dass es als mindestens eine aktive Komponente eine Verbindung der Formel I gemäss Anspruch 1 enthält.

11. Mittel gemäss Anspruch 10, dadurch gekennzeichnet, dass es als mindestens eine aktive Komponente eine Verbindung der Formel I gemäss Anspruch 2 enthält.

12. Mittel zur Bekämpfung von Mikroorganismen gemäss Anspruch 10, dadurch gekennzeichnet, dass es als mindestens eine aktive Komponente eine Verbindung der Formel I gemäss einem der Ansprüche 3 bis 9 enthält.

13. Mittel nach Anspruch 10, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

14. Mittel nach Anspruch 13, dadurch gekennzeichnet, dass es 0,1 bis 95 % eines Wirkstoffs der Formel I, 99,8 bis 5 % eines festen oder flüssigen Zusatzstoffes und 0,1 bis 25 % eines Tensids enthält.

15. Verfahren zur Herstellung eines wie in Anspruch 10 beanspruchten agrochemischen Mittel, dadurch gekennzeichnet, dass man mindestens eine gemäss Anspruch 1 definierte Verbindung der

Formel I mit geeigneten festen oder flüssigen Zusatzstoffen und Tensiden innig vermischt.

16. Verfahren zur Bekämpfung oder Verhütung eines Befalls von Kulturpflanzen durch phytopathogene Schädlinge, dadurch gekennzeichnet, dass man eine gemäss Anspruch 1 definierte Verbindung der Formel I auf die Pflanze oder deren Standort appliziert.

**Claims** (for the Contracting States : GB, BE, DE, FR, IT, LU, NL, SE, CH, LI)

1. Compounds of the general formula I

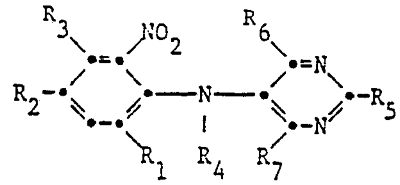

(I)

wherein each of

$R_1$ and $R_2$ independently of the other is $NO_2$ or $CF_3$, or one of the two is hydrogen ;

$R_3$ is hydrogen or halogen ;

$R_4$ is hydrogen or the —C(O)R′ group in which R′ is $C_1$-$C_4$alkyl which is unsubstituted or is substituted by halogen, $C_1$-$C_3$alkoxy or by $C_1$-$C_3$alkylthio ; each of

$R_5$, $R_6$ and $R_7$ independently of the others is hydrogen, halogen, nitro, cyano, thiocyano, mercapto, $C_1$-$C_6$alkyl, $C_1$-$C_6$haloalkyl, $C_1$-$C_6$alkoxy, $C_1$-$C_6$alkoxy which is substituted by halogen, nitro, cyano, $C_1$-$C_3$alkoxy, $C_1$-$C_3$alkylthio and/or by $N(C_1$-$C_4alkyl)_2$, or is $C_3$-$C_6$cycloalkoxy, $C_3$-$C_6$alkenyloxy, $C_3$-$C_6$alkynyloxy, $C_1$-$C_6$alkylthio, $C_3$-$C_6$alkenylthio, $C_1$-$C_6$alkylsulphonyl, $C_1$-$C_6$alkylsulphoxyl, $C_3$-$C_6$-alkenyl, $C_3$-$C_6$haloalkenyl, $C_3$-$C_6$alkynyl, $C_3$-$C_6$haloalkynyl or the $N(C_1$-$C_4alkyl)_2$ group, and $R_5$ may additionally be a phenyl or benzyl group which are both unsubstituted or substituted by from one to three substituents selected from halogen, nitro, cyano, $C_1$-$C_4$alkyl, $C_1$-$C_3$haloalkyl, $N(C_1$-$C_3alkyl)_2$ and/or $C_1$-$C_3$alkoxy.

2. Compounds of formula I according to claim 1, characterised in that

$R_1$ is $NO_2$ or $CF_3$ ;

$R_2$ is $NO_2$ or $CF_3$ ;

$R_3$ is hydrogen or halogen ;

$R_4$ is hydrogen or the —C(O)R′ group in which R′ is $C_1$-$C_4$alkyl which is unsubstituted or is substituted by halogen, $C_1$-$C_3$alkoxy or by $C_1$-$C_3$alkylthio ; each of

$R_5$, $R_6$ and $R_7$ independently of the others is hydrogen, halogen, nitro, cyano, thiocyano, mercapto, $C_1$-$C_6$alkyl, $C_1$-$C_6$haloalkyl, $C_1$-$C_6$alkoxy, $C_1$-$C_6$alkoxy which is substituted by halogen, nitro, cyano, $C_1$-$C_3$alkoxy, $C_1$-$C_3$alkylthio and/or by $N(C_1$-$C_4alkyl)_2$, or is $C_3$-$C_6$cycloalkoxy, $C_3$-$C_6$alkenyloxy, $C_3$-$C_6$alkynyloxy, $C_1$-$C_6$alkylthio, $C_3$-$C_6$alkenylthio, $C_1$-$C_6$alkylsulphonyl, $C_1$-$C_6$alkylsulphoxyl or the $N(C_1$-$C_4alkyl)_2$ group, and $R_5$ may additionally be a phenyl or benzyl group which is unsubstituted or substituted by from one to three substituents selected from halogen, nitro, cyano, $C_1$-$C_4$alkyl, $C_1$-$C_3$haloalkyl, $N(C_1$-$C_3alkyl)_2$ and/or $C_1$-$C_3$alkoxy.

3. Compounds of formula I according to claim 2, characterised in that $R_1$ is nitro, $R_2$ is trifluoromethyl, $R_3$ is chlorine, $R_4$ is hydrogen or the —C(O)R′ group in which R′ is $C_1$-$C_4$alkyl which is unsubstituted or is substituted by halogen, $C_1$-$C_3$alkoxy or by $C_1$-$C_3$alkylthio, $R_5$ is hydrogen, halogen, cyano, thiocyano, $C_1$-$C_6$alkyl, $C_1$-$C_3$haloalkyl, $C_1$-$C_6$alkoxy, $C_1$-$C_3$haloalkoxy, $C_1$-$C_3$alkylthio-($C_1$-$C_3$alkoxy), $C_1$-$C_3$alkylthio, $C_1$-$C_3$alkoxy which is substituted by $N(C_1$-$C_2alkyl)_2$, or is $C_3$-$C_4$alkenyloxy, $C_3$-$C_4$alkenylthio, $C_1$-$C_3$alkylsulphonyl, $C_1$-$C_3$alkylsulphoxyl, $N(C_1$-$C_3alkyl)_2$, or is a phenyl or benzyl group which are both unsubstituted or substituted by from one to three substituents selected from fluorine, chlorine, bromine, nitro, cyano, methoxy, $C_1$-$C_4$alkyl, dimethylamino or $CF_3$ ; and each of $R_6$ and $R_7$ independently of the other is hydrogen, halogen, cyano, thiocyano, $C_1$-$C_6$alkyl, $C_1$-$C_3$haloalkyl, $C_1$-$C_6$alkoxy, $C_1$-$C_3$alkoxy which is substituted by halogen, nitro, $C_1$-$C_3$alkoxy, $C_1$-$C_3$alkylthio or by $N(C_1$-$C_4alkyl)_2$, or is $C_1$-$C_4$alkylthio, $C_1$-$C_3$alkylsulphonyl, $C_1$-$C_3$alkylsulphoxyl, $C_3$-$C_4$alkenyloxy, $C_3$-$C_4$alkenylthio, $C_3$-$C_4$alkynyloxy, $C_3$-$C_4$alkynylthio or $N(C_1$-$C_3alkyl)_2$.

4. Compounds of formula I according to claim 3, characterised in that $R_4$ is hydrogen and the other substituents are as defined in claim 3.

5. Compounds of formula I according to claim 2, characterised in that $R_1$ is nitro, $R_2$ is trifluoromethyl, $R_3$ is hydrogen, $R_4$ is hydrogen or the —C(O)R′ group in which R′ is $C_1$-$C_4$alkyl which is unsubstituted or is substituted by halogen, $C_1$-$C_3$alkoxy or by $C_1$-$C_3$alkylthio, $R_5$ is hydrogen, halogen, cyano, thiocyano, $C_1$-$C_6$alkyl, $C_1$-$C_3$haloalkyl, $C_1$-$C_6$alkoxy, $C_1$-$C_3$haloalkoxy, $C_1$-$C_3$alkylthio-($C_1$-$C_3$alkoxy), $C_1$-$C_3$alkylthio, $C_1$-$C_3$-alkoxy which is substituted by $N(C_1$-$C_2alkyl)_2$, or is $C_3$-$C_4$alkenyloxy, $C_3$-$C_4$alkenylthio, $C_1$-$C_3$alkylsulphonyl, $C_1$-$C_3$alkylsulphoxyl, $N(C_2$-$C_3alkyl)_2$, or is a phenyl or benzyl group which are both are unsubstituted or substituted by from one to three substituents selected from fluorine,

chlorine, bromine, nitro, cyano, methoxy, $C_1$-$C_4$alkyl, dimethylamino or $CF_3$ ; and each of $R_6$ and $R_7$ independently of the other is hydrogen, halogen, cyano, thiocyano, $C_1$-$C_6$alkyl, $C_1$-$C_3$haloalkyl, $C_1$-$C_6$alkoxy, $C_1$-$C_3$alkoxy which is substituted by halogen, nitro, $C_1$-$C_3$alkoxy, $C_1$-$C_3$alkylthio or by N($C_1$-$C_4$alkyl)$_2$, or is $C_1$-$C_4$alkylthio, $C_1$-$C_3$alkylsulphonyl, $C_1$-$C_3$alkylsulphoxyl, $C_3$-$C_4$alkenyloxy, $C_3$-$C_4$alkenylthio, $C_3$-$C_4$alkynyloxy, $C_3$-$C_4$alkynylthio or N($C_1$-$C_3$alkyl)$_2$.

6. Compounds of formula I according to claim 5, characterised in that $R_4$ is hydrogen and the other substituents are as defined in claim 5.

7. Compounds of formula I according to claim 2, characterised in that $R_1$ is trifluoromethyl, $R_2$ is nitro, $R_3$ is hydrogen, $R_4$ is hydrogen or the —C(O)R′ group in which R′ is $C_1$-$C_4$alkyl which is unsubstituted or is substituted by halogen, $C_1$-$C_3$alkoxy or by $C_1$-$C_3$alkylthio, $R_5$ is hydrogen, halogen, cyano, thiocyano, $C_1$-$C_6$alkyl, $C_1$-$C_3$haloalkyl, $C_1$-$C_6$alkoxy, $C_1$-$C_3$haloalkoxy, $C_1$-$C_3$alkylthio-($C_1$-$C_3$alkoxy), $C_1$-$C_3$alkylthio, $C_1$-$C_3$alkoxy which is substituted by N($C_1$-$C_2$alkyl)$_2$, or is $C_3$-$C_4$alkenyloxy, $C_3$-$C_4$alkenylthio, $C_1$-$C_3$alkylsulphonyl, $C_1$-$C_3$alkylsulphoxyl, N($C_1$-$C_3$alkyl)$_2$, or is a phenyl or benzyl group which are both unsubstituted or substituted by from one to three substituents selected from fluorine, chlorine, bromine, nitro, cyano, methoxy, $C_1$-$C_4$alkyl, dimethylamino of $CF_3$ ; and each of $R_6$ and $R_7$ independently of the other is hydrogen, halogen, cyano, thiocyano, $C_1$-$C_6$alkyl, $C_1$-$C_3$haloalkyl, $C_1$-$C_6$alkoxy, $C_1$-$C_3$alkoxy which is substituted by halogen, nitro, $C_1$-$C_3$alkoxy, $C_1$-$C_3$alkylthio or by N($C_1$-$C_4$alkyl)$_2$, or is $C_1$-$C_4$alkylthio, $C_1$-$C_3$alkylsulphonyl, $C_1$-$C_3$alkylsulphoxyl, $C_3$-$C_4$alkenyloxy, $C_3$-$C_4$alkenylthio, $C_3$-$C_4$alkynyloxy, $C_3$-$C_4$alkynylthio or N($C_1$-$C_3$alkyl)$_2$.

8. Compounds of formula I according to claim 7, characterised in that $R_4$ is hydrogen and the other substituents are as defined in claim 7.

9. A compound of formula I according to claim 1, selected from the group consisting of :
N-(3′-chloro-2′,6′-dinitro-4′-trifluoromethylphenyl)-5-amino-4-chloro-6-methoxypyrimidine (No. 1.1),
N-(3′-chloro-2′,6′-dinitro-4′-trifluoromethylphenyl)-5-amino-4,6-dimethylthiopyrimidine (No. 1.2),
N-(3′-chloro-2′,6′-dinitro-4′-trifluoromethylphenyl)-5-amino-4,6-ditrifluoroethoxypyrimidine (No. 1.3),
N-(3′-chloro-2′,6′-dinitro-4′-trifluoromethylphenyl)-5-amino-4,6-dichloropyrimidine (No. 1.4),
N-(3′-chloro-2′,6′-dinitro-4′-trifluoromethylphenyl)-5-amino-4-chloro-6-methylmercaptopyrimidine (No. 1.184),
N-3′-chloro-2′,6′-dinitro-4′-trifluoromethylphenyl)-5-amino-4-chloro-2-methyl-6-methoxypyrimidine (No. 1.205),
N-(3′-chloro-2′,6′-dinitro-4′-trifluoromethylphenyl)-5-amino-2-chloro-4-methoxypyrimidine (No. 1.51),
N-(3′-chloro-2′,6′-dinitro-4′-trifluoromethylphenyl)-5)-amino-2,4-dichloropyrimidine (No. 1.49),
N-(3′-chloro-2′,6′-dinitro-4′-trifluoromethylphenyl)-5-amino-2-chloro-4-allyloxypyrimidine (No. 1.210),
N-(3′-chloro-2′,6′-dinitro-4′-trifluoromethylphenyl)-5-amino-2-chloro-4-allylmercaptopyrimidine (No. 1.57),
N-(3′-chloro-2′,6′-dinitro-4′-trifluoromethylphenyl)-5-amino-2-chloro-4-propargyloxypyrimidine (No. 1.211),
N-(3′-chloro-2′,6′-dinitro-4′-trifluoromethylphenyl)-5-amino-4,6-dichloro-2-methylpyrimidine (No. 1.96),
N-(3′-chloro-2′,6′-dinitro-4′-trifluoromethylphenyl)-5-amino-4,6-dichloro-2-phenylpyrimidine (No. 1.125),
N-(2′,6′-dinitro-4′-trifluoromethylphenyl)-5-amino-4,6-dichloropyrimidine (No. 2.4),
N-(2′,4′-dinitro-6′-trifluoromethylphenyl)-5-amino-4,6-dichloropyrimidine (No. 3.4),
N-(2′,4′,6′-trinitrophenyl)-5-amino-4,6-dichloropyrimidine (No. 7.1),
N-(2′-nitro-4′-trifluoromethylphenyl)-5-amino-4,6-dichloropyrimidine (No. 8.1).

10. A process for the preparation of compounds of formula I, characterised in that a compound of formula II

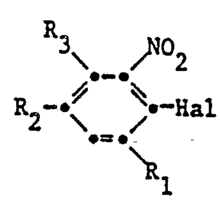

(II)

is reacted in the presence of a base with a pyrimidine derivative of formula III

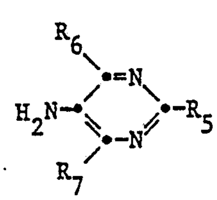

(III)

to give a compound of formula I′

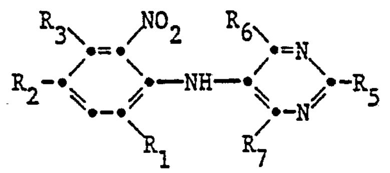

(I′)

and, to obtain N-acylated derivatives, the latter is N-acylated with a reactive derivative of the carboxylic acid of formula IV

$R_4COOH$ (IV),

in which formulae the substituents $R_1$ to $R_7$ are as defined for formula I and hal is halogen.

11. A composition for controlling or preventing attack by pests, characterised in that it contains a compound of formula I as at least one active ingredient, together with conventional adjuvants and carriers.

12. A method of controlling or preventing attack of cultivated plants by phytopathogenic pests, characterised in that a compound of formula I is applied to the plan or to the locus thereof.

**Claims** (for the Contracting State AT)

1. A process for the preparation of compounds of formula I

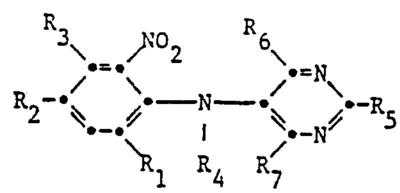

(I)

wherein each of

$R_1$ and $R_2$ independently of the other is $NO_2$ or $CF_3$, or one of the two is hydrogen ;

$R_3$ is hydrogen or halogen ;

$R_4$ is hydrogen or the —C(O)R′ group in which R′ is $C_1$-$C_4$alkyl which is unsubstituted or is substituted by halogen, $C_1$-$C_3$alkoxy or by $C_1$-$C_3$alkylthio ; each of

$R_5$, $R_6$ and $R_7$ independently of the others is hydrogen, halogen, nitro, cyano, thiocyano, mercapto, $C_1$-$C_6$alkyl, $C_1$-$C_6$haloalkyl, $C_1$-$C_6$alkoxy, $C_1$-$C_6$alkoxy which is substituted by halogen, nitro, cyano, $C_1$-$C_3$alkoxy, $C_1$-$C_3$alkylthio and/or by $N(C_1$-$C_4alkyl)_2$, or is $C_3$-$C_6$cycloalkoxy, $C_3$-$C_6$alkenyloxy, $C_3$-$C_6$alkynyloxy, $C_1$-$C_6$alkylthio, $C_3$-$C_6$alkenylthio, $C_1$-$C_6$alkylsulphonyl, $C_1$-$C_6$alkylsulphoxyl, $C_3$-$C_6$alkenyl, $C_3$-$C_6$haloalkenyl, $C_3$-$C_6$alkynyl, $C_3$-$C_6$haloalkynyl or the $N(C_1$-$C_4alkyl)_2$ group, and $R_5$ may additionally be a phenyl or benzyl group which are both unsubstituted or substituted by from one to three substituents selected from halogen, nitro, cyano, $C_1$-$C_4$alkyl, $C_1$-$C_3$haloalkyl, $N(C_1$-$C_3alkyl)_2$ and/or $C_1$-$C_3$alkoxy, characterised in that a compound of formula II

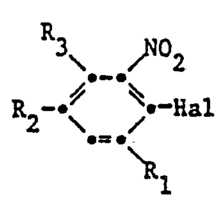

(II)

is reacted in the presence of a base with a pyrimidine derivative of formula III

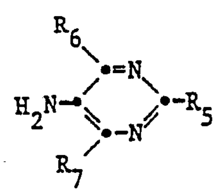

(III)

to give a compound of formula I'

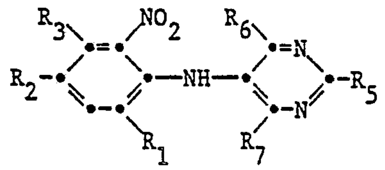

(I')

and, to obtain N-acylated derivatives, the latter is N-acylated with a reactive derivative of the carboxylic acid of formula IV

$$R_4COOH \qquad (IV),$$

in which formulae the substituents $R_1$ to $R_7$ are as defined for formula I and Hal is halogen.

2. A process according to claim 1, for the preparation of compounds of formula I wherein $R_1$ is $NO_2$ or $CF_3$ ; $R_2$ is $NO_2$ or $CF_3$ ; $R_3$ is hydrogen or halogen ; $R_4$ is hydrogen or the —C(O)R' group in which R' is $C_1$-$C_4$alkyl which is unsubstituted or is substituted by halogen, $C_1$-$C_3$alkoxy or by $C_1$-$C_3$alkylthio ; each of $R_5$, $R_6$ and $R_7$ independently of the others is hydrogen, halogen, nitro, cyano, thiocyano, mercapto, $C_1$-$C_6$alkyl, $C_1$-$C_6$haloalkyl, $C_1$-$C_6$alkoxy, $C_1$-$C_6$alkoxy which is substituted by halogen, nitro, cyano, $C_1$-$C_3$alkoxy, $C_1$-$C_3$alkylthio and/or by N($C_1$-$C_4$alkyl)$_2$, or is $C_3$-$C_6$cycloalkoxy, $C_3$-$C_6$alkenyloxy, $C_3$-$C_6$alkynyloxy, $C_1$-$C_6$alkylthio, $C_3$-$C_6$alkenylthio, $C_1$-$C_6$alkylsulphonyl, $C_1$-$C_6$alkylsulphoxyl or the N($C_1$-$C_4$alkyl)$_2$ group, and $R_5$ may additionally be a phenyl or benzyl group which is unsubstituted or substituted by from one to three substituents selected from halogen, nitro, cyano, $C_1$-$C_4$alkyl, $C_1$-$C_3$haloalkyl, N($C_1$-$C_3$alkyl)$_2$ and/or $C_1$-$C_3$alkoxy.

3. A process according to claim 2, for the preparation of compounds of formula I wherein $R_1$ is nitro, $R_2$ is trifluoromethyl, $R_3$ is chlorine, $R_4$ is hydrogen or the —C(O)R' group in which R' is $C_1$-$C_4$alkyl which is unsubstituted or is substituted by halogen, $C_1$-$C_3$alkoxy or by $C_1$-$C_3$alkylthio, $R_5$ is hydrogen, halogen, cyano, thiocyano, $C_1$-$C_6$alkyl, $C_1$-$C_3$haloalkyl, $C_1$-$C_6$alkoxy, $C_1$-$C_3$haloalkoxy, $C_1$-$C_3$alkylthio-($C_1$-$C_3$alkoxy), $C_1$-$C_3$alkylthion, $C_1$-$C_3$alkoxy which is substituted by N($C_1$-$C_2$alkyl)$_2$, or is $C_3$-$C_4$alkenyloxy, $C_3$-$C_4$alkenylthio, $C_1$-$C_3$alkylsulphonyl, $C_1$-$C_3$alkylsulphoxyl, N($C_1$-$C_3$alkyl)$_2$, or is a phenyl or benzyl group which are both unsubstituted or substituted by from one to three substituents selected from fluorine, chlorine, bromine, nitro, cyano, methoxy, $C_1$-$C_4$alkyl, dimethylamino of $CF_3$ ; and each of $R_6$ and $R_7$ independently of the other is hydrogen, halogen, cyano, thiocyano, $C_1$-$C_6$alkyl, $C_1$-$C_3$haloalkyl, $C_1$-$C_6$alkoxy, $C_1$-$C_3$alkoxy which is substituted by halogen, nitro, $C_1$-$C_3$alkoxy, $C_1$-$C_3$alkylthio or by N($C_1$-$C_4$alkyl)$_2$, or is $C_1$-$C_4$alkylthio, $C_1$-$C_3$alkylsulphonyl, $C_1$-$C_3$alkylsulphoxyl, $C_3$-$C_4$alkenyloxy, $C_3$-$C_4$alkenylthio, $C_3$-$C_4$alkynyloxy, $C_3$-$C_4$alkynylthio or N($C_1$-$C_3$alkyl)$_2$.

4. A process according to claim 3, for the preparation of compounds of formula I wherein $R_4$ is hydrogen and the other substituents are as defined in claim 3.

5. A process according to claim 2, for the preparation of compounds of formula I according to claim 1, wherein $R_1$ is nitro, $R_2$ is trifluoromethyl, $R_3$ is hydrogen, $R_4$ is hydrogen or the —C(O)R' group in which R' is $C_1$-$C_4$alkyl which is unsubstituted or is substituted by halogen, $C_1$-$C_3$alkoxy or by $C_1$-$C_3$alkylthio, $R_5$ is H, halogen, cyano, thiocyano, $C_1$-$C_6$alkyl, $C_1$-$C_3$haloalkyl, $C_1$-$C_6$alkoxy, $C_1$-$C_3$haloalkoxy, $C_1$-$C_3$alkylthio-($C_1$-$C_3$alkoxy), $C_1$-$C_3$alkylthio, $C_1$-$C_3$alkoxy which is substituted by N($C_1$-$C_2$alkyl)$_2$, or is $C_3$-$C_4$alkenyloxy, $C_3$-$C_4$alkenylthio, $C_1$-$C_3$alkylsulphonyl, $C_1$-$C_3$alkylsulphoxyl, N($C_1$-$C_3$alkyl)$_2$, or is a phenyl or benzyl group which are both unsubstituted or substituted by from one to three substituents selected from fluorine, chlorine, bromine, nitro, cyano, methoxy, $C_1$-$C_4$alkyl, dimethylamino or $CF_3$ ; and each of $R_6$ and $R_7$ independently of the other is hydrogen, halogen, cyano, thiocyano, $C_1$-$C_6$alkyl, $C_1$-$C_3$haloalkyl, $C_1$-$C_6$alkoxy, $C_1$-$C_3$alkoxy which is substituted by halogen, nitro, $C_1$-$C_3$alkoxy, $C_1$-$C_3$alkylthio or by N($C_1$-$C_4$alkyl)$_2$, or is $C_1$-$C_4$alkylthio, $C_1$-$C_3$alkylsulphonyl, $C_1$-$C_3$alkylsulphoxyl, $C_3$-$C_4$alkenyloxy, $C_3$-$C_4$alkenylthio, $C_3$-$C_4$alkynyloxy, $C_3$-$C_4$alkynylthio or N($C_1$-$C_3$alkyl)$_2$.

6. A process according to claim 5, for the preparation of compounds of formula I wherein $R_4$ is hydrogen and the other substituents are as defined in claim 5.

7. A process according to claim 2, for the preparation of compounds of formula I wherein $R_1$ is trifluoromethyl, $R_2$ is nitro, $R_3$ is hydrogen, $R_4$ is hydrogen or the —C(O)R' group in which R' is $C_1$-$C_4$alkyl which is unsubstituted or is substituted by halogen, $C_1$-$C_3$alkoxy or by $C_1$-$C_3$alkylthio, $R_5$ is hydrogen, halogen, cyano, thiocyano, $C_1$-$C_6$alkyl, $C_1$-$C_3$haloalkyl, $C_1$-$C_6$alkoxy, $C_1$-$C_3$haloalkoxy, $C_1$-$C_3$alkylthio-($C_1$-$C_3$alkoxy), $C_1$-$C_3$alkylthio, $C_1$-$C_3$alkoxy wich is substituted by N($C_1$-$C_2$alkyl)$_2$, or is $C_3$-$C_4$alkenyloxy, $C_3$-$C_4$alkenylthio, $C_1$-$C_4$alkylsulphonyl, $C_1$-$C_3$alkylsulphoxyl, N($C_1$-$C_3$alkyl)$_2$, or is a phenyl or benzyl group which are both unsubstituted or substituted by from one to three substituents selected from fluorine, chlorine, bromine, nitro, cyano, methoxy, $C_1$-$C_4$alkyl, dimethylamino of $CF_3$ ; and each of $R_6$ and $R_7$ independently of the other is hydrogen, halogen, cyano, thiocyano, $C_1$-$C_6$alkyl, $C_1$-$C_3$haloalkyl, $C_1$-$C_6$alkoxy, $C_1$-$C_3$alkoxy which is substituted by halogen, nitro, $C_1$-$C_3$alkoxy, $C_1$-$C_3$alkylthio or by N($C_1$-

$C_4$alkyl)$_2$, or is $C_1$-$C_4$alkylthio, $C_1$-$C_3$alkylsulphonyl, $C_1$-$C_3$alkylsulphoxyl, $C_1$-$C_4$alkenyloxy, $C_3$-$C_4$alkenylthio, $C_3$-$C_4$alkynyloxy, $C_3$-$C_4$alkynylthio of $N(C_1$-$C_3$alkyl$)_2$.

8. A process according to claim 7, for the preparation of compounds of formula I wherein $R_4$ is hydrogen and the other substituents are as defined in claim 7.

9. A process according to claim 1, for the preparation of a compound of formula I selected from the group consisting of :

N-(3'-chloro-2',6'-dinitro-4'-trifluoromethylphenyl)-5-amino-4-chloro-6-methoxypyrimidine (No. 1.1),
N-(3'-chloro-2',6'-dinitro-4'-trifluoromethylphenyl)-5-amino-4,6-dimethylthiopyrimidine (No. 1.2),
N-(3'-chloro-2',6'-dinitro-4'-trifluoromethylphenyl)-5-amino-4,6-ditrifluoroethoxypyrimidine (No. 1.3),
N-(3'-chloro-2',6'-dinitro-4'-trifluoromethylphenyl)-5-amino-4,6-dichloropyrimidine (No. 1.4),
N-(3'-chloro-2',6'-dinitro-4'-trifluoromethylphenyl)-5-amino-4-chloro-6-methylmercaptopyrimidine (No. 1.184),
N-(3'-chloro-2',6'-dinitro-4'-trifluoromethylphenyl)-5-amino-4-chloro-2-methyl-6-methoxypyrimidine (No. 1.205),
N-(3'-chloro-2',6'-dinitro-4'-trifluoromethylphenyl)-5-amino-2-chloro-4-methoxypyrimidine (No. 1.51),
N-(3'-chloro-2',6'-dinitro-4'-trifluoromethylphenyl)-5-amino-2,4-dichloropyrimidine (No. 1.49),
N-(3'-chloro-2',6'-dinitro-4'-trifluoromethylphenyl)-5-amino-2-chloro-4-allyloxypyrimidine (No. 1.210),
N-(3'-chloro-2',6'-dinitro-4'-trifluoromethylphenyl)-5-amino-2-chloro-4-allylmercaptopyrimidine (No. 1.57),
N-(3'-chloro-2',6'-dinitro-4'-trifluoromethylphenyl)-5-amino-2-chloro-4-propargyloxypyrimidine (No. 1.211),
N-(3'-chloro-2',6'-dinitro-4'-trifluoromethylphenyl)-5-amino-4,6-dichloro-2-methylpyrimidine (No. 1.96),
N-(3'-chloro-2',6'-dinitro-4'-trifluoromethylphenyl)-5-amino-4,6-dichloro-2-phenylpyrimidine (No. 1.125),
N-(2',6'-dinitro-4'-trifluoromethylphenyl)-5-amino-4,6-dichloropyrimidine (No. 2.4),
N-(2',4'-dinitro-6'-trifluoromethylphenyl)-5-amino-4,6-dichloropyrimidine (No. 3.4),
N-(2',4',6'-trinitrophenyl)-5-amino-4,6-dichloropyrimidine (No. 7.1),
N-(2'-nitro-4'-trifluoromethylphenyl)-5-amino-4,6-dichloropyrimidine (No. 8.1).

10. A composition for controlling or preventing attack by pests, characterised in that it contains a compound of formula I according to claim 1 as at least one active ingredient.

11. A composition according to claim 10, characterised in that it contains a compound of formula I according to claim 2 as at least one active ingredient.

12. A composition for controlling microorganisms according to claim 10, characterised in that it contains a compound of formula I according to any one of claims 3 to 9 as at least one active ingredient.

13. A composition according to claim 10, characterised in that it contains from 0.1 to 99 % of a compound of formula I, from 99.9 to 1 % of a solid or liquid adjuvant and from 0 to 25 % of a surfactant.

14. A composition according to claim 13, characterised in that it contains from 0.1 to 95 % of a compound of formula I, from 99.8 to 5 % of a solid or liquid adjuvant and from 0.1 to 25 % of a surfactant.

15. A process for the preparation of an agrochemical composition as claimed in claim 10, characterised in that at least one compound of formula I defined according to claim 1 is homogeneously mixed with suitable solid or liquid adjuvants and surfactants.

16. A method of controlling or preventing attack of cultivated plants by phytopathogenic pests, characterised in that a compound of formula I defined according to claim 1 is applied to the plant or to the locus thereof.

**Revendications** (pour les Etats contractants : GB, BE, DE, FR, IT, LU, NL, SE, CH, LI)

1. Composés de formule générale I

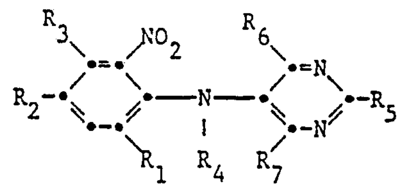

(I)

dans laquelle

$R_1$ et $R_2$ représentent chacun, indépendamment l'un de l'autre, $NO_2$ ou $CF_3$, ou bien l'un des deux symboles représente l'hydrogène ;

$R_3$ représente l'hydrogène ou un halogène ;

$R_4$ représente l'hydrogène ou le groupe —C(O)R' dans lequel R' représente un groupe alkyle en C 1-C 4 non substitué ou substitué par des halogènes ; des groupes alcoxy en C 1-C 3 ou alkylthio en C 1-C 3 ;

$R_5$, $R_6$ et $R_7$ représentent chacun, indépendamment les uns des autres, l'hydrogène, un halogène, un groupe nitro, cyano, thiocyano, mercapto, alkyle en C 1-C 6, halogénoalkyle en C 1-C 6, alcoxy en C 1-C 6, alcoxy en C 1-C 6 substitué par des halogènes, des groupes nitro, cyano, alcoxy en C 1-C 3, alkylthio en C 1-C 3 et/ou N-(alkyle en C 1-C $4)_2$, un groupe cycloalcoxy en C 3-C 6, alcényloxy en C 3-C 6, alcynyloxy en C 3-C 6, alkylthio en C 1-C 6, alcénylthio en C 3-C 6, alkylsulfonyle en C 1-C 6, alkylsulfoxyle en C 1-C 6, alcényle en C 3-C 6, halogénoalcényle en C 3-C 6, alcynyle en C 3-C 6, halogénoalcynyle en C 3-C 6 ou le groupe N-(alkyle en C 1-C $4)_2$, et $R_5$ peut en outre représenter un groupe phényle ou benzyle non substitué ou mono- à tri-substitué par des halogènes, des groupes nitro, cyano, alkyle en C 1-C 4, halogénoalkyle en C 1-C 3, N-(alkyle en C 1-C $3)_2$ et/ou alcoxy en C 1-C 3.

2. Composés de formule I selon la revendication 1, caractérisés en ce que :

$R_1$ représente $NO_2$ ou $CF_3$ ;

$R_2$ représente $NO_2$ ou $CF_3$ ;

$R_3$ représente l'hydrogène ou un halogène ;

$R_4$ représente l'hydrogène ou le groupe —C(O)R' dans lequel R' représente un groupe alkyle en C 1-C 4 non substitué ou substitué par des halogènes, des groupes alcoxy en C 1-C 3 ou alkylthio en C 1-C 3 ;

$R_5$, $R_6$ et $R_7$ représentent chacun, indépendamment les uns des autres, l'hydrogène, un halogène, un groupe nitro, cyano, thiocyano, mercapto, alkyle en C 1-C 6, halogénoalkyle en C 1-C 6, alcoxy en C 1-C 6, alcoxy en C 1-C 6 substitué par des halogènes, des groupes nitro, cyano, alcoxy en C 1-C 3, alkylthio en C 1-C 3 et/ou N-(alkyle en C 1-C $4)_2$, un groupe cycloalcoxy en C 3-C 6, alcényloxy en C 3-C 6, alcynyloxy en C 3-C 6, alkylthio en C 1-C 6, alcénylthio en C 3-C 6, alkylsulfonyle en C 1-C 6, alkylsulfoxyle en C 1-C 6 ou le groupe N-(alkyle en C 1-C $4)_2$ et $R_5$ peut en outre représenter un groupe phényle ou benzyle non substitué ou mono- à tri-substitué par des halogènes, des groupes nitro, cyano, alkyle en C 1-C 4, halogénoalkyle en C 1-C 3, N-(alkyle en C 1-C $3)_2$ et/ou alcoxy en C 1-C 3.

3. Composés de formule I selon la revendication 2, caractérisés en ce que : $R_1$ représente un groupe nitro, $R_2$ un groupe trifluorométhyle, $R_3$ le chlore, $R_4$ l'hydrogène ou le groupe —C(O)R' dans lequel R' représente un grupe alkyle en C 1-C 4 non substitué ou substitué par des halogènes, des groupes alcoxy en C 1-C 3 ou alkylthio en C 1-C 3 ;

$R_5$ représente l'hydrogène, un halogène, un groupe cyano, thiocyano, alkyle en C 1-C 6, halogénoalkyle en C 1-C 3, alcoxy en C 1-C 6, halogénoalcoxy en C 1-C 3, (alkylthio en C 1-C 3)-alcoxy en C 1-C 3, alkylthio en C 1-C 3, alcoxy en C 1-C 3 substitué par un groupe N-(alkyle en C 1-C $2)_2$, un groupe alcényloxy en C 3-C 4, alcénylthio en C 3-C 4, alkylsulfonyle en C 1-C 3, alkylsulfoxyle en C 1-C 3, N-(alkyle en C 1-C $3)_2$ ou un groupe phényle ou benzyle, chacun non substitué ou mono- à trisubstitué par le fluor, le chlore, le brome, des groupes nitro, cyano, méthoxy, alkyle en C 1-C 4, diméthylamino ou $CF_3$ ; et $R_6$ et $R_7$ représentent chacun, indépendamment l'un de l'autre, l'hydrogène, un halogène, un groupe cyano, thiocyano, alkyle en C 1-C 6, halogénoalkyle en C 1-C 3, alcoxy en C 1-C 6, alcoxy en C 1-C 3 substitué par des halogènes, des groupes nitro, alcoxy en C 1-C 3, alkylthio en C 1-C 3 ou N-(alkyle en C 1-C $4)_2$, un groupe alkylthio en C 1-C 4, alkylsulfonyle en C 1-C 3, alkylsulfoxyle en C 1-C 3, alcényloxy en C 3-C 4, alcénylthio en C 3-C 4, alcynyloxy en C 3-C 4, alcynylthio en C 3-C 4 ou N-(alkyle en C 1-C $3)_2$.

4. Composés de formule I selon la revendication 3, caractérisés en ce que $R_4$ représente l'hydrogène et les autres symboles ont les significations indiquées dans la revendication 3.

5. Composés de formule I selon la revendication 2, caractérisés en ce que $R_1$ représente un groupe nitro, $R_2$ un groupe trifluorométhyle, $R_3$ l'hydrogène, $R_4$ l'hydrogène ou le groupe —C(O)R' dans lequel R' représente un groupe alkyle en C 1-C 4 non substitué ou substitué par des halogènes, des groupes alcoxy en C 1-C 3 ou alkylthio en C 1-C 3 ;

$R_5$ représente l'hydrogène, un halogène, un groupe cyano, thiocyano, alkyle en C 1-C 6, halogénoalkyle en C 1-C 3, alcoxy en C 1-C 6, halogénoalcoxy en C 1-C 3, (alkylthio en C 1-C 3)-alcoxy en C 1-C 3, alkylthio en C 1-C 3, alcoxy en C 1-C 3 substitué par N-(alkyle en C 1-C $2)_2$, alcényloxy en C 3-C 4, alcénylthio en C 3-C 4, alkylsulfonyle en C 1-C 3, alkylsulfoxyle en C 1-C 3, N-(alkyle en C 1-C $3)_2$ ou un groupe phényle ou benzyle chacun non substitué ou mono- à tri-substitué par le fluor, le chlore, le brome, des groupes nitro, cyano, méthoxy, alkyle en C 1-C 4, diméthylamino ou $CF_3$ ; et $R_6$ et $R_7$ représentent chacun, indépendamment l'un de l'autre, l'hydrogène, un halogène, un groupe cyano, thiocyano, alkyle en C 1-C 6, halogénoalkyle en C 1-C 3, alcoxy en C 1-C 6, alcoxy en C 1-C 3 substitué par des halogènes, des groupes nitro, alcoxy en C 1-C 3, alkylthio en C 1-C 3 ou N-(alkyle en C 1-C $4)_2$, un groupe alkylthio en C 1-C 4, alkylsulfonyle en C 1-C 3, alkylsulfoxyle en C 1-C 3, alcényloxy en C 3-C 4, alcénylthio en C 3-C 4, alcynyloxy en C 3-C 4, alcynylthio en C 3-C 4 ou N-(alkyle en C 1-C $3)_2$.

6. Composés de formule I selon la revendication 5, caractérisés en ce que $R_4$ représente l'hydrogène et les autres symboles ont les significations indiquées dans la revendication 5.

7. Composés de formule I selon la revendication 2, caractérisés en ce que $R_1$ représente un groupe trifluorométhyle, $R_2$ un groupe nitro, $R_3$ l'hydrogène, $R_4$ l'hydrogène ou le groupe —C(O)R' dans lequel R' représente un groupe alkyle en C 1-C 4 non substitué ou substitué par des halogènes, des groupes alcoxy en C 1-C 3 ou alkylthio en C 1-C 3 ;

$R_5$ représente l'hydrogène, un halogène, un groupe cyano, thiocyano, alkyle en C 1-C 6, halogénoalkyle en C 1-C 3, alcoxy en C 1-C 6, halogénoalcoxy en C 1-C 3, (alkylthio en C 1-C 3)-alcoxy en C 1-C 3, alkylthio en C 1-C 3, alcoxy en C 1-C 3 substitué par N-(alkyle en C 1-C $2)_2$, alcényloxy en C 3-C 4, alcénylthio en C 3-C 4, alkylsulfonyle en C 1-C 3, alkylsulfoxyle en C 1-C 3, N-(alkyle en C 1-C $3)_2$ ou un

groupe phényle ou benzyle chacun non substitué ou mono- à tri-substitué par le fluor, le chlore, le brome, des groupes nitro, cyano, méthoxy, alkyle en C 1-C 4, diméthylamino ou $CF_3$ ; et $R_6$ et $R_7$ représentent chacun, indépendamment l'un de l'autre, l'hydrogène, un halogène, un groupe cyano, thiocyano, alkyle en C 1-C 6, halogénoalkyle en C 1-C 3, alcoxy en C 1-C 6, alcoxy en C 1-C 3 substitué par des halogènes, des groupes nitro, alcoxy en C 1-C 3, alkylthio en C 1-C 3 ou N-(alkyle en C 1-C 4)$_2$, un groupe alkylthio en C 1-C 4, alkylsulfonyle en C 1-C 3, alkylsulfoxyle en C 1-C 3, alcényloxy en C 3-C 4, alcénylthio en C 3-C 4, alcynyloxy en C 3-C 4, alcynylthio en C 3-C 4 ou N-(alkyle en C 1-C 3)$_2$.

8. Composés de formule I selon la revendication 7, caractérisés en ce que $R_4$ représente l'hydrogène et les autres symboles ont les significations indiquées dans la revendication 7.

9. Un composé de formule I selon la revendication 1, choisi dans le groupe suivant :
N-(3'-chloro-2',6'-dinitro-4'-trifluorométhylphényl)-5-amino-4-fluoro-6-méthoxypyrimidine (n° 1.1),
N-(3'-chloro-2',6'-dinitro-4'-trifluorométhylphényl)-5-amino-4,6-diméthylthiopyrimidine (n° 1.2),
N-(3'-chloro-2',6'-dinitro-4'-trifluorométhylphényl)-5-amino-4,6-ditrifluoréthoxypyrimidine (n° 1.3),
N-(3'-chloro-2',6'-dinitro-4'-trifluorométhylphényl)-5-amino-4,6-dichloropyrimidine (n° 1.4),
N-(3'-chloro-2',6'-dinitro-4'-trifluorométhylphényl)-5-amino-4-chloro-6-méthylmercaptopyrimidine (n° 1.184),
N-(3'-chloro-2',6'-dinitro-4'-trifluorométhylphényl)-5-amino-4-chloro-2-méthyl-6-méthoxypyrimidine (n° 1.205),
N-(3-chloro-2',6'-dinitro-4'-trifluorométhylphényl)-5-amino-2-chloro-4-méthoxypyrimidine (n° 1.51),
N-(3'-chloro-2',6'-dinitro-4'-trifluorométhylphényl)-5-amino-2,4-dichloropyrimidine (n° 1.49),
N-(3'-chloro-2',6'-dinitro-4'-trifluorométhylphényl)-5-amino-2-chloro-4-allyloxypyrimidine (n° 1.210),
N-(3'-chloro-2',6'-dinitro-4'-trifluorométhylphényl)-5-amino-2-chloro-4-allylmercaptopyrimidine (n° 1.57),
N-(3'-chloro-2',6'-dinitro-4'-trifluorométhylphényl)-5-amino-2-chloro-4-propargyloxypyrimidine (n° 1.211),
N-(3'-chloro-2',6'-dinitro-4'-trifluorométhylphényl)-5-amino-4,6-dichloro-2-méthylpyrimidine (n° 1.96),
N-(3'-chloro-2',6'-dinitro-4'-trifluorométhylphényl)-5-amino-4,6-dichloro-2-phénylpyrimidine (n° 1.125),
N-(2',6'-dinitro-4'-trifluorométhylphényl)-5-amino-4,6-dichloropyrimidine (n° 2.4),
N-(2',4-dinitro-6'-trifluorométhylphényl-(5-amino-4,6-dichloropyrimidine (n° 3.4),
N-(2',4',6'-trinitrophényl)-5-amino-4,6-dichloropyrimidine (n° 7.1),
N-(2'-nitro-4'-trifluorométhylphényl)-5-amino-4,6-dichloropyrimidine (n° 8.1).

10. Procédé de préparation des composés de formule I, caractérisé en ce que l'on fait réagir un composé de formule II

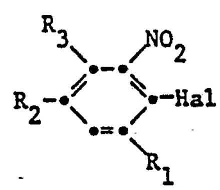

(II)

en présence d'une base avec un dérivé de la pyrimidine de formule III

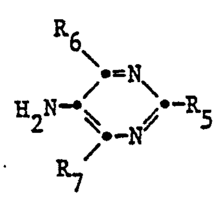

(III)

ce qui donne un composé de formule I'

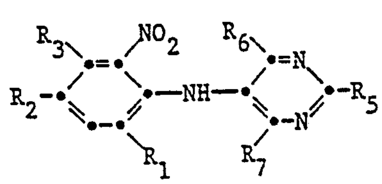

(I')

qu'on soumet, pour préparer les dérivés N-acylés, à N-acylation par un dérivé réactif de l'acide carboxylique de formule IV

$R_4COOH$ (IV)

les symboles $R_1$ à $R_7$ ayant les significations indiquées en référence à la formule I et Hal représentant un halogène.

11. Produit pour combattre ou prévenir une infestation par des parasites, caractérisé en ce qu'il contient au moins un composant actif consistant en un composé de formule I avec des additifs et véhicules usuels.

12. Procédé pour combattre ou prévenir une infestation de végétaux cultivés par des parasites phytopathogènes, caractérisé en ce que l'on applique sur la plante ou son habitat un composé de formule I.

**Revendications** (pour l'Etat contractant AT)

1. Procédé de préparation des composés de formule I

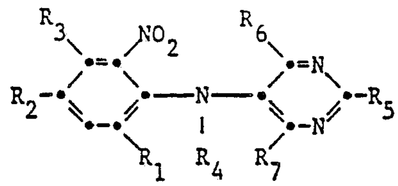

(I)

dans laquelle

$R_1$ et $R_2$ représentent chacun, indépendamment l'un de l'autre, $NO_2$ ou $CF_3$, ou bien l'un des deux symboles représente l'hydrogène ;

$R_3$ représente l'hydrogène ou un halogène ;

$R_4$ représente l'hydrogène ou le groupe —C(O)R' dans lequel R' représente un groupe alkyle en C 1-C 4 non substitué ou substitué par des halogènes, des groupes alcoxy en C 1-C 3 ou alkylthio en C 1-C 3 ;

$R_5$, $R_6$ et $R_7$ représentent chacun, indépendamment les uns des autres, l'hydrogène, un halogène, un groupe nitro, cyano, thiocyano, mercapto, alkyle en C 1-C 6, halogénoalkyle en C 1-C 6, alcoxy en C 1-C 6, alcoxy en C 1-C 6 substitué par des halogènes, des groupes nitro, cyano, alcoxy en C 1-C 3, alkylthio en C 1-C 3 et/ou N-(alkyle en C 1-C 4)$_2$, un groupe cycloalcoxy en C 3-C 6, alcényloxy en C 3-C 6, alcynyloxy en C 3-C 6, alkylthio en C 1-C 6, alcénylthio en C 3-C 6, alkylsulfonyle en C 1-C 6, alkylsulfoxyle en C 1-C 6, alcényle en C 3-C 6, halogénoalcényle en C 3-C 6, alcynyle en C 3-C 6, halogénoalcynyle en C 3-C 6 ou le groupe N-(alkyle en C 1-C 4)$_2$, et $R_5$ peut en outre représenter un groupe phényle ou benzyle non substitué ou mono- à tri-substitué par des halogènes, des groupes nitro, cyano, alkyle en C 1-C 4, halogénoalkyle en C 1-C 3, N-(alkyle en C 1-C 3)$_2$ et/ou alcoxy en C 1-C 3,

caractérisé en ce que l'on fait réagir un composé de formule II

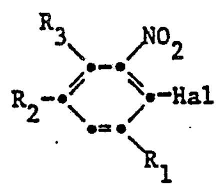

(II)

en présence d'une base avec un dérivé de la pyrimidine de formule III

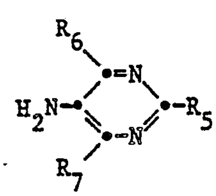

(III)

ce qui donne un composé de formule I'

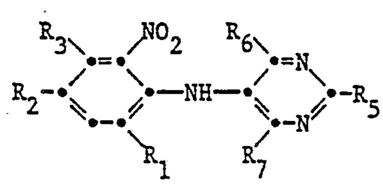

(I')

qu'on soumet, pour préparer les dérivés N-acylés, à N-acylation par un dérivé réactif de l'acide carboxylique de formule IV

$$R_4COOH \quad (IV)$$

les symboles $R_1$ à $R_7$ ayant les significations indiquées en référence à la formule I et Hal réprésentant un halogène.

2. Procédé selon la revendication 1, pour la préparation des composés de formule I dans laquelle

$R_1$ représente $NO_2$ ou $CF_3$ ;

$R_2$ représente $NO_2$ ou $CF_3$ ;

$R_3$ représente l'hydrogène ou un halogène ;

$R_4$ représente l'hydrogène ou le groupe —C(O)R' dans lequel R' représente un groupe alkyle en C 1-C 4 non substitué ou substitué par des halogènes, des groupes alkoxy en C 1-C 3 ou alkylthio en C 1-C 3 ;

$R_5$, $R_6$ et $R_7$ représentent chacun, indépendamment les uns des autres, l'hydrogène, un halogène, un groupe nitro, cyano, thiocyano, mercapto, alkyle en C 1-C 6, halogénoalkyle en C 1-C 6, alcoxy en C 1-C 6, alcoxy en C 1-C 6 substitué par des halogènes, des groupes nitro, cyano, alcoxy en C 1-C 3, alkylthio en C 1-C 3 et/ou N-(alkyle en C 1-C $4)_2$, un groupe cycloalcoxy en C 3-C 6, alcényloxy en C 3-C 6, alcynyloxy en C 3-C 6, alkylthio en C 1-C 6, alcénylthio en C 3-C 6, alkylsulfonyle en C 1-C 6, alkylsulfoxyle en C 1-C 6 ou le groupe N-(alkyle en C 1-C $4)_2$ et $R_5$ peut en outre représenter un groupe phényle ou benzyle non substitué ou mono- à tri-substitué par des halogènes, des groupes nitro, cyano, alkyle en C 1-C 4, halogénoalkyle en C 1-C 3, N-(alkyle en C 1-C $3)_2$ et/ou alcoxy en C 1-C 3.

3. Procédé selon la revendication 2, pour la préparation des composés de formule I dans laquelle $R_1$ représente un groupe nitro, $R_2$ un groupe trifluorométhyle, $R_3$ le chlore, $R_4$ l'hydrogène ou le groupe —C(O)R' dans lequel R' représente un groupe alkyle en C 1-C 4 non substitué ou substitué par des halogènes, des groupes alcoxy en C 1-C 3 ou alkylthio en C 1-C 3 ; $R_5$ représente l'hydrogène, un halogène, un groupe cyano, thiocyano, alkyle en C 1-C 6, halogénoalkyle en C 1-C 3, alcoxy en C 1-C 6, halogénoalcoxy en C 1-C 3, (alkylthio en C 1-C 3)-alcoxy en C 1-C 3, alkylthio en C 1-C 3, alcoxy en C 1-C 3 substitué par un groupe N-(alkyle en C 1-C $2)_2$, un groupe alcényloxy en C 3-C 4, alcénylthio en C 3-C 4, alkylsulfonyle en C 1-C 3, alkylsulfoxyle en C 1-C 3, N-(alkyle en C 1-C $3)_2$ ou un groupe phényle ou benzyle, chacun non substitué ou mono- à trisubstitué par le fluor, le chlore, le brome, des groupes nitro, cyano, méthoxy, alkyle en C 1-C 4, diméthylamino ou $CF_3$ ; et $R_6$ et $R_7$ représentent chacun, indépendamment l'un de l'autre, l'hydrogène, un halogène, un groupe cyano, thiocyano, alkyle en C 1-C 6, halogénoalkyle en C 1-C 3, alcoxy en C 1-C 6, alcoxy en C 1-C 3 substitué par des halogènes, des groupes nitro, alcoxy en C 1-C 3, alkylthio en C 1-C 3 ou N-(alkyle en C 1-C $4)_2$, un groupe alkylthio en C 1-C 4, alkylsulfonyle en C 1-C 3, alkylsulfoxyle en C 1-C 3, alcényloxy en C 3-C 4, alcénylthio en C 3-C 4, alcynyloxy en C 3-C 4, alcynylthio en C 3-C 4 ou N-(alkyle en C 1-C $3)_2$.

4. Procédé selon la revendication 3, pour la préparation de composés de formule I dans laquelle $R_4$ représente l'hydrogène et les autres symboles ont les significations indiquées dans la revendication 3.

5. Procédé selon la revendication 2, pour la préparation de composés de formule I selon la revendication 1, dans laquelle $R_1$ représente un groupe nitro, $R_2$ un groupe trifluorométhyle, $R_3$ l'hydrogène, $R_4$ l'hydrogène ou le groupe —C(O)R' dans lequel R' représente un groupe alkyle en C 1-C 4 non substitué ou substitué par des halogènes, des groupes alcoxy en C 1-C 3 ou alkylthio en C 1-C 3 ;

$R_5$ représente l'hydrogène, un halogène, un groupe cyano, thiocyano, alkyle en C 1-C 6, halogénoalkyle en C 1-C 3, alcoxy en C 1-C 6, halogénoalcoxy en C 1-C 3, (alkylthio en C 1-C 3)-alcoxy en C 1-C 3, alkylthio en C 1-C 3, alcoxy en C 1-C 3 substitué par N-(alkyle en C 1-C $2)_2$, alcényloxy en C 3-C 4, alcénylthio en C 3-C 4, alkylsulfonyle en C 1-C 3, alkylsulfoxyle en C 1-C 3, N-(alkyle en C 1-C $3)_2$ ou un groupe phényle ou benzyle chacun non substitué ou mono- à tri-substitué par le fluor, le chlore, le brome, des groupes nitro, cyano, méthoxy, alkyle en C 1-C 4, diméthylamino ou $CF_3$ ; et $R_6$ et $R_7$ représentent chacun, indépendamment l'un de l'autre, l'hydrogène, un halogène, un groupe cyano, thiocyano, alkyle en C 1-C 6, halogénoalkyle en C 1-C 3, alcoxy en C 1-C 6, alcoxy en C 1-C 3 substitué par des halogènes, des groupes nitro, alcoxy en C 1-C 3, alkylthio en C 1-C 3 ou N-(alkyle en C 1-C $4)_2$, un groupe alkylthio en C 1-C 4, alkylsulfonyle en C 1-C 3, alkylsulfoxyle en C 1-C 3, alcényloxy en C 3-C 4, alcénylthio en C 3-C 4, alcynyloxy en C 3-C 4, alcynylthio en C 3-C 4 ou N-(alkyle en C 1-C $3)_2$.

6. Procédé selon la revendication 5, pour la préparation de composés de formule I dans laquelle $R_4$ représente l'hydrogène et les autres symboles ont les significations indiquées dans la revendication 5.

7. Procédé selon la revendication 2, pour la préparation de composés de formule I dans laquelle $R_1$ représente un groupe trifluorométhyle, $R_2$ un groupe nitro, $R_3$ l'hydrogène, $R_4$ l'hydrogène ou le groupe —C(O)R' dans lequel R' représente un groupe alkyle en C 1-C 4 non substitué ou substitué par des halogènes, des groupes alcoxy en C 1-C 3 ou alkylthio en C 1-C 3 ; $R_5$ représente l'hydrogène, un halogène, un groupe cyano, thiocyano, alkyle en C 1-C 6, halogénoalkyle en C 1-C 3, alcoxy en C 1-C 6, halogénoalcoxy en C 1-C 3, (alkylthio en C 1-C 3)-alcoxy en C 1-C 3, alkylthio en C 1-C 3, alcoxy en C 1-C 3 substitué par N-(alkyle en C 1-C $2)_2$, alcényloxy en C 3-C 4, alcénylthio en C 3-C 4, alkylsulfonyle en C 1-C 3, alkylsulfoxyle en C 1-C 3, N-(alkyle en C 1-C $3)_2$ ou un groupe phényle ou benzyle chacun non substitué ou mono- à tri-substitué par le fluor, le chlore, le brome, des groupes nitro, cyano, méthoxy, alkyle en C 1-C 4, diméthylamino ou $CF_3$ ; et $R_6$ et $R_7$ représentent chacun, indépendamment l'un de

l'autre, l'hydrogène, un halogène, un groupe cyano, thiocyano, alkyle en C 1-C 6, halogénoalkyle en C 1-C 3, alcoxy en C 1-C 6, alcoxy en C 1-C 3 substitué par des halogènes, des groupes nitro, alcoxy en C 1-C 3, alkylthio en C 1-C 3 ou N-(alkyle en C 1-C 4)$_2$, un groupe alkylthio en C 1-C 4, alkylsulfonyle en C 1-C 3, alkylsulfoxyle en C 1-C 3, alcényloxy en C 3-C 4, alcénylthio en C 3-C 4, alcynyloxy en C 3-C 4, alcynylthio en C 3-C 4 ou N-(alkyle en C 1-C 3)$_2$.

8. Procédé selon la revendication 7, pour la préparation de composés de formule I dans laquelle $R_4$ représente l'hydrogène et les autres symboles ont les significations indiquées dans la revendication 7.

9. Procédé selon la revendication 1, pour la préparation d'un composé de formule I choisi dans le groupe suivant :

N-(3'-chloro-2',6'-dinitro-4'-trifluorométhylphényl)-5-amino-4-fluoro-6-méthoxypyrimidine (n° 1.1),
N-(3'-chloro-2',6'-dinitro-4'-trifluorométhylphényl)-5-amino-4,6-diméthylthiopyrimidine (n° 1.2),
N-(3'-chloro-2',6'-dinitro-4'-trifluorométhylphényl)-5-amino-4,6-ditrifluoréthoxypyrimidine (n° 1.3),
N-(3'-chloro-2',6'-dinitro-4'-trifluorométhylphényl)-5-amino-4,6-dichloropyrimidine (n° 1.4),
N-(3'-chloro-2',6'-dinitro-4'-trifluorométhylphényl)-5-amino-4-chloro-6-méthylmercaptopyrimidine (n° 1.184),
N-(3'-chloro-2',6'-dinitro-4'-trifluorométhylphényl)-5-amino-4-chloro-2-méthyl-6-méthoxypyrimidine (n° 1.205),
N-(3-chloro-2',6'-dinitro-4'-trifluorométhylphényl)-5-amino-2-chloro-4-méthoxypyrimidine (n° 1.51),
N-(3'-chloro-2',6'-dinitro-4'-trifluorométhylphényl)-5-amino-2,4-dichloropyrimidine (n° 1.49),
N-(3'-chloro-2',6'-dinitro-4'-trifluorométhylphényl)-5-amino-2-chloro-4-allyloxypyrimidine (n° 1.210),
N-(3'-chloro-2',6'-dinitro-4'-trifluorométhylphényl)-5-amino-2-chloro-4-allylmercaptopyrimidine (n° 1.57),
N-(3'-chloro-2',6'-dinitro-4'-trifluorométhylphényl)-5-amino-2-chloro-4-propargyloxypyrimidine (n° 1.211),
N-(3'-chloro-2',6'-dinitro-4'-trifluorométhylphényl)-5-amino-4,6-dichloro-2-méthylpyrimidine (n° 1.96),
N-(3'-chloro-2',6'-dinitro-4'-trifluorométhylphényl)-5-amino-4,6-dichloro-2-phénylpyrimidine (n° 1.125),
N-(2',6'-dinitro-4'-trifluorométhylphényl)-5-amino-4,6-dichloropyrimidine (n° 2.4),
N-(2',4-dinitro-6'-trifluorométhylphényl)-5-amino-4,6-dichloropyrimidine (n° 3.4),
N-(2',4',6'-trinitrophényl)-5-amino-4,6-dichloropyrimidine (n° 7.1),
N-(2'-nitro-4'-trifluorométhylphényl-5-amino-4,6-dichloropyrimidine (n° 8.1).

10. Produit pour combattre ou prévenir une infestation par des parasites, caractérisé en ce qu'il contient au moins un composant actif consistant en un composé de formule I selon la revendication 1.

11. Produit selon la revendication 10, caractérisé en ce qu'il contient au moins un composant actif consistant en un composé de formule I selon la revendication 2.

12. Produit pour combattre les micro-organismes selon la revendication 10, caractérisé en ce qu'il contient au moins un composant actif consistant en un composé de formule I selon l'une des revendications 3 à 9.

13. Produit selon la revendication 10, caractérisé en ce qu'il contient de 0,1 à 99 % d'une substance active de formule I, de 99,9 à 1 % d'un additif solide ou liquide et de 0 à 25 % d'un agent tensioactif.

14. Produit selon la revendication 13, caractérisé en ce qu'il contient de 0,1 à 95 % d'une substance active de formule I, de 99,8 à 5 % d'un additif solide ou liquide et de 0,1 à 25 % d'un agent tensioactif.

15. Procédé de préparation d'un produit agrochimique tel que revendiqué dans la revendication 10, caractérisé en ce que l'on mélange intimement au moins un composé de formule I définie dans la revendication 1 avec des additifs solides ou liquides et des agents tensioactifs appropriés.

16. Procédé pour combattre ou prévenir une infestation de végétaux cultivés par des parasites phytopathogènes, caractérisé en ce que l'on applique un composé de formule I défini dans la revendication 1 sur la plante ou son habitat.